# EUROPEAN PATENT SPECIFICATION

(11) **EP 1 109 790 B1**
(45) Date of publication and mention of the grant of the patent: **14.05.2003**
(21) Application number: 99946526.3
(22) Date of filing: 27.08.1999
(51) Int. Cl.: C07D 249/12, C07D 401/04, C07D 405/04, C07D 409/04, A61K 31/4196

(54) **1, 2, 4-TRIAZOLE-3-THIONE COMPOUNDS**
1,2,4-TRIAZOL-3-THION VERBINDUNGEN
COMPOSES 1,2,4-TRIAZOLE-3-THIONE

(30) Priority: 01.09.1998 SE 9802937
(43) Date of publication of application: 27.06.2001
(73) Proprietor: AstraZeneca AB, 151 85 Södertälje (SE)
(72) Inventor: BAXTER, Andrew, Loughborough, Leics. LE11 5RH (GB); BENNION, Colin, Loughborough, Leics. LE11 5RH (GB); CAGE, Peter, Loughborough, Leics. LE11 5RH (GB); KINDON, Nicholas, Loughborough, Leics. LE11 5RH (GB); MORTIMORE, Michael, Loughborough, Leics. LE11 5RH (GB); ROBERTS, Bryan, Loughborough, Leics. LE11 5RH (GB)
(86) International application number: SE9901469
(87) International publication number: WO00012489

(56) References cited:
- WO-A1-98/04135
- STN INTERNATIONAL, File CAPLUS, CAPLUS Accession No. 1967:37843, Document No. 66:37843, DURANT GRAHAM J., "The reaction of benzoyl isothiocyanate with hydrazine derivatives. I. Reaction with some alkyl hydrazines", XP002943900 & J. CHEM. SOC. C, no. 1, 1967, pages 92 - 94

## Description

The present invention relates to certain 1,2,4-triazole-3-thione compounds, processes for their preparation, pharmaceutical compositions containing them and their use in therapy.

WO 98/04135 discloses a class of substituted triazoles having the general formula 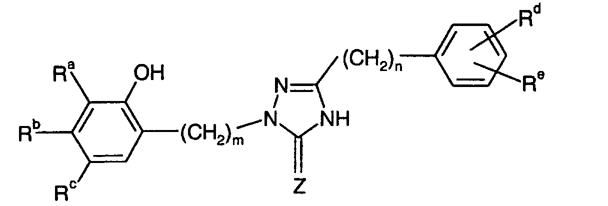 wherein Z is O or S; R^{a}, R^{b} and R^{c} each are independently selected from hydrogen, halogen, OH, CF₃, NO₂ or 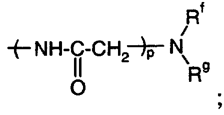 provided R^{c} is not hydrogen; and when R^{a} and R^{b} are hydrogen, R^{c} may be a heterocyclic moiety selected from the group consisting of imidazol-1-yl, morpholinomethyl, N-methylimidazol-2-yl and pyridin-2-yl; R^{d} and R^{e} each are independently selected from hydrogen, halogen, CF₃, NO₂ or imidazol-1-yl; m, n and p each are independently selected from an integer of 0 or 1; and R^{f} and R^{g} each are independently hydrogen; C₁-C₄ alkyl; or R^{f} and R^{g}, taken together with the nitrogen atom to which they are attached, is a heterocyclic moiety selected from the group consisting of N-methylpiperazine, morpholine, thiomorpholine, N-benzylpiperazine and imidazolinone. The substituted triazoles are said to act as potassium channel modulators, having application in the treatment of disorders such as ischaemia. Further substituted traizoles are also disclosed in J.Chem Soc., 1967 p92-94.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C) and Cys-Cys (C-C) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

In accordance with the present invention, there is therefore provided a compound of general formula 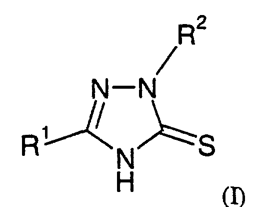 wherein R¹ represents a phenyl or naphthyl group, or a 5- or 6-membered heterocyclic aromatic group containing at least one heteroatom selected from nitrogen, oxygen and sulphur, each group being optionally substituted by one or more substituents independently selected from halogen atoms, nitro, cyano, hydroxyl, C₁-C₆ alkyl, C₁-C₆ alkoxy, C₁-C₆ alkoxycarbonyl, C₁-C₆ alkylthio, phenyl, phenoxy, phenylcarbonyl and trifluoromethyl groups; and
R² represents a C₁₋C₆ alkylphenyl group optionally substituted by one or more substituents independently selected from halogen atoms, hydroxyl, nitro, cyano, C₁-C₆ alkyl, C₁-C₆ alkoxy, phenoxy and trifluoromethyl groups,
with the provisos that:
- when R² represents an unsubstituted C₁ alkylphenyl group, then R¹ is not a phenyl, 4-chlorophenyl or 3-pyridinyl group,
- when R² represents an unsubstituted C₂ alkylphenyl group, then R¹ is not a phenyl group, and
- when R² represents a substituted C₁ alkylphenyl group, then it is not substituted by a hydroxyl group in the 2-position of the phenyl ring;
or a pharmaceutically acceptable salt or solvate thereof.

In the present specification, unless otherwise indicated, an alkyl group or alkyl moiety in an alkoxy, alkoxycarbonyl or alkylthio group may be linear or branched.

Preferably R¹ in formula (I) above represents a phenyl or naphthyl group, or a 5- or 6-membered heterocyclic aromatic group containing one, two or three heteroatoms independentry selected from nitrogen, oxygen and sulphur (e.g. a furanyl, thienyl, pyridinyl or pyrimidinyl group), each group being optionally substituted by one, two, three or four substituents independently selected from halogen atoms (e.g. fluorine, chlorine, bromine or iodine), nitro, cyano, hydroxyl, C₁-C₆ alkyl (e.g. methyl, ethyl, propyl, isopropyl, butyl, isobutyl, tertiary-butyl, pentyl or hexyl), C₁-C₆ alkoxy (e.g. methoxy, ethoxy, propoxy, butoxy, pentoxy or hexoxy), C₁-C₆ alkoxycarbonyl (e.g. methoxycarbonyl or ethoxycarbonyl), C₁-C₆ alkylthio (e.g. methylthio or ethylthio), phenyl, phenoxy, phenylcarbonyl and trifluoromethyl groups.

More preferably, R¹ represents a phenyl or naphthyl group, or a 5- or 6-membered heterocyclic aromatic group containing one or two heteroatoms independently selected from nitrogen, oxygen and sulphur, each group being optionally substituted by one or two substituents independently selected from halogen atoms, nitro, cyano, hydroxyl, C₁-C₄ alkyl, C₁-C₄ alkoxy, C₁-C₄ alkoxycarbonyl, C₁-C₄ alkylthio, phenyl, phenoxy, phenylcarbonyl and trifluoromethyl groups.

Still more preferably, R¹ represents a phenyl or naphthyl group, or a 5- or 6-membered heterocyclic aromatic group containing one or two heteroatoms independently selected from nitrogen, oxygen and sulphur, each group being optionally substituted by one or two substituents independently selected from halogen atoms, nitro, cyano, C₁-C₃ alkyl (especially methyl or isopropyl), methoxy, methoxycarbonyl, methylthio, phenyl, phenoxy, phenylcarbonyl and trifluoromethyl groups.

It is preferred if, in formula (I) above, R² represents a C₁₋C₆ alkylphenyl group optionally substituted by one, two, three or four substituents independently selected from halogen atoms (e.g. fluorine, chlorine, bromine or iodine), hydroxyl, nitro, cyano, C₁-C₆ alkyl (e.g. methyl, ethyl, propyl, isopropyl, butyl, isobutyl, tertiary-butyl, pentyl or hexyl), C₁-C₆ alkoxy (e.g. methoxy, ethoxy, propoxy, butoxy, pentoxy or hexoxy), phenoxy and trifluoromethyl groups. The optional substituents are preferably attached to the phenyl moiety.

More preferably, R² represents a C₁₋C₄ alkylphenyl group optionally substituted by one or two substituents independently selected from halogen atoms, hydroxyl, nitro, cyano, C₁-C₄ alkyl, C₁-C₄ alkoxy, phenoxy and trifluoromethyl groups.

Most preferably, R² represents a C₁₋C₃ alkylphenyl (especially methylphenyl, ethylphenyl or propylphenyl) group optionally substituted by one or two substituents independently selected from halogen atoms, hydroxyl, nitro, cyano, C₁-C₄ alkyl (especially methyl and tertiary-butyl), methoxy, phenoxy and trifluoromethyl groups.

Especially preferred compounds of the invention include:
5-(2,5-Dichlorophenyl)-1,2-dihydro-2-(phenylmethyl)-3*H*-1,2,4-triazole-3-thione,
2-[1,2-Dihydro-1-(phenylmethyl)-5-thioxo-3*H*-1,2,4-triazol-3-yl]benzoic acid, methyl ester,
4-[1,2-Dihydro-1-(phenylmethyl)-5-thioxo-3*H*-1,2,4-triazol-3-yl]benzoic acid, methyl ester,
5-(5-Chloro-2-thienyl)-1,2-dihydro-2-(phenylmethyl)-3*H*-1,2,4-triazole-3-thione,
5-(3-Chlorophenyl)-1,2-dihydro-2-(phenylmethyl)-3*H*-1,2,4-triazole-3-thione,
1,2-Dihydro-5-(2-nitrophenyl)-2-(phenylmethyl)-3*H*-1,2,4-triazole-3-thione,
5-(4-Cyanophenyl)-1,2-dihydro-2-(phenylmethyl)-3*H*-1,2,4-triazole-3-thione,
5-(2,3-Dichlorophenyl)-1,2-dihydro-2-(phenylmethyl)-3*H*-1,2,4-triazole-3-thione,
5-(2-Bromophenyl)-1,2-dihydro-2-(phenylmethyl)-3*H*-1,2,4-triazole-3-thione,
1,2-Dihydro-2-(phenylmethyl)-5-[3-(trifluoromethyl)phenyl]-3*H*-1,2,4-triazole-3-thione,
5-(2-Furanyl)-1,2-dihydro-2-(phenylmethyl)-3*H*-1,2,4-triazole-3-thione,
5-(2-Bromo-5-methoxyphenyl)-1,2-dihydro-2-(phenylmethyl)-3*H*-1,2,4-triazole-3-thione,
5-(2,5-Dichloro-3-thienyl)-1,2-dihydro-2-(phenylmethyl)-3*H*-1,2,4-triazole-3-thione,
1,2-Dihydro-2-(phenylmethyl)-5-(4-pyridinyl)-3*H*-1,2,4-triazole-3-thione,
1,2-Dihydro-2-(phenylmethyl)-5-(4-phenylphenyl)-3*H*-1,2,4-triazole-3-thione,
5-(2-Chloro-3-pyridinyl)-1,2-dihydro-2-(phenylmethyl)-3*H*-1,2,4-triazole-3-thione,
5-(2-Fluorophenyl)-1,2-dihydro-2-(phenylmethyl)-3*H*-1,2,4-triazole-3-thione,
1,2-Dihydro-5-(2-methoxyphenyl)-2-(phenylmethyl)-3*H*-1,2,4-triazole-3-thione,
1,2-Dihydro-5-(2-methylphenyl)-2-(phenylmethyl)-3*H*-1,2,4-triazole-3-thione,
5-(3-Chloro-2-thienyl)-1,2-dihydro-2-(phenylmethyl)-3*H*-1,2,4-triazole-3-thione,
1,2-Dihydro-2-(phenylmethyl)-5-[4-(trifluoromethyl)phenyl]-3*H*-1,2,4-triazole-3-thione,
5-(2-Chloro-4-fluorophenyl)-1,2-dihydro-2-(phenylmethyl)-3*H*-1,2,4-triazole-3-thione,
1,2-Dihydro-5-(4-methoxyphenyl)-2-(phenylmethyl)-3*H*-1,2,4-triazole-3-thione,
1,2-Dihydro-5-(4-methylphenyl)-2-(phenylmethyl)-3*H*-1,2,4-triazole-3-thione,
5-(3,4-Dichlorophenyl)-1,2-dihydro-2-(phenylmethyl)-3*H*-1,2,4-triazole-3-thione,
5-(4-Bromophenyl)-1,2-dihydro-2-(phenylmethyl)-3*H*-1,2,4-triazole-3-thione,
5-(2,4-Dichlorophenyl)-1,2-dihydro-2-(phenylmethyl)-3*H*-1,2,4-triazole-3-thione,
5-(2-Chlorophenyl)-1,2-dihydro-2-(phenylmethyl)-3*H*-1,2,4-triazole-3-thione,
5-(3,5-Dichlorophenyl)-1,2-dihydro-2-(phenylmethyl)-3*H*-1,2,4-triazole-3-thione,
1,2-Dihydro-5-(3-phenoxyphenyl)-2-(phenylmethyl)-3*H*-1,2,4-triazole-3-thione,
1,2-Dihydro-2-(phenylmethyl)-5-(2-thienyl)-3*H*-1,2,4-triazole-3-thione,
2-[(3-Chlorophenyl)methyl]-5-(2,4-dichlorophenyl)-1,2-dihydro-3*H*-1,2,4-triazole-3-thione,
5-(2,4-Dichlorophenyl)-1,2-dihydro-2-[(3-methoxyphenyl)methyl]-3*H*-1,2,4-triazole-3-thione,
5-(2,4-Dichlorophenyl)-1,2-dihydro-2-[(3-phenoxyphenyl)methyl]-3*H*-1,2,4-triazole-3-thione,
1,2-Dihydro-2-[(3-hydroxyphenyl)methyl]-5-phenyl-3*H*-1,2,4-triazole-3-thione,
5-(2-Chloro-6-fluorophenyl)-1,2-dihydro-2-(phenylmethyl)-3*H*-1,2,4-triazole-3-thione,
1,2-Dihydro-5-(1-naphthalenyl)-2-(phenylmethyl)-3*H*-1,2,4-triazole-3-thione,
1,2-Dihydro-5-[2-(methylthio)phenyl]-2-(phenylmethyl)-3*H*-1,2,4-triazole-3-thione,
1,2-Dihydro-5-[2-(phenoxy)phenyl]-2-(phenylmethyl)-3*H*-1,2,4-triazole-3-thione,
1,2-Dihydro-5-(3-(phenylcarbonyl)phenyl)-2-(phenylmethyl)-3*H*-1,2,4-triazole-3-thione,
1,2-Dihydro-5-(1-bromo-2-naphthalenyl)-2-(phenylmethyl)-3*H*-1,2,4-triazole-3-thione,
5-(2-Chlorophenyl)-2-[(3-chlorophenyl)methyl]-1,2-dihydro-3*H*-1,2,4-triazole-3-thione,
5-(2,4-Dichlorophenyl)-1,2-dihydro-2-[(3-methylphenyl)methyl]-3*H*-1,2,4-triazole-3-thione,
5-(2,4-Dichlorophenyl)-1,2-dihydro-2-(2-phenylethyl)-3*H*-1,2,4-triazole-3-thione,
2-[(4-Chlorophenyl)methyl]-5-(2,4-dichlorophenyl)-1,2-dihydro-3*H*-1,2,4-triazole-3-thione,
5-(4-Bromophenyl)-2-[(3-chlorophenyl)methyl]-1,2-dihydro-3*H*-1,2,4-triazole-3-thione,
2-[(3-Chlorophenyl)methyl]-5-(4-methylphenyl)-1,2-dihydro-3*H*-1,2,4-triazole-3-thione,
2-[(3-Chlorophenyl)methyl]-5-(4-methoxyphenyl)-1,2-dihydro-3*H*-1,2,4-triazole-3-thione,
2-[(3-Chlorophenyl)methyl]-5-[4-(1-methylethyl)phenyl]-1,2-dihydro-3*H*-1,2,4-triazole-3-thione,
5-(2-Chlorophenyl)-1,2-dihydro-2-(4-methylphenyl)methyl-3*H*-1,2,4-triazole-3-thione,
5-(2-Chlorophenyl)-1,2-dihydro-2-[(4-( 1,1-dimethylethyl)phenyl)methyl]-3*H*-1,2,4-triazole-3-thione,
5-(2-Chlorophenyl)-1,2-dihydro-2-[(3-phenyloxy)phenylmethyl]-3*H*-1,2,4-triazole-3-thione,
5-(2-Chlorophenyl)-1,2-dihydro-2-[[4-(trifluoromethyl)phenyl]methyl]-3*H*-1,2,4-triazole-3-thione,
5-(2-Chlorophenyl)-1,2-dihydro-2-[(4-(methoxyphenyl)methyl]-3*H*-1,2,4-triazole-3-thione,
2-[(3-Chlorophenyl)methyl]-5-(4-phenoxyphenyl)-1,2-dihydro-3*H*-1,2,4-triazole-3-thione,
2-[(3-Chlorophenyl)methyl]-5-(3-pyridinyl)-1,2-dihydro-3*H*-1,2,4-triazole-3-thione,
2-[(3-Chlorophenyl)methyl ]-5-phenyl-1,2-dihydro-3*H*-1,2,4-triazole-3-thione,
5-(2-Chlorophenyl)-1,2-dihydro-2-[(3-fluorophenyl)methyl]-3*H*-1,2,4-triazole-3-thione, 5-(2-Chlorophenyl)-1,2-dihydro-2-[(2-iodophenyl)methyl]-3*H*-1,2,4-triazole-3-thione,
5-(2-Chlorophenyl)-1,2-dihydro-2-[(2,5-dimethylphenyl)methyl]-3*H*-1,2,4-triazole-3-thione,
5-(2-Chlorophenyl)-1,2-dihydro-2-[(2,5-difluorophenyl)methyl]-3*H*-1,2,4-triazole-3-thione,
2-[(3-Chlorophenyl)methyl]-5-(4-nitrophenyl)-1,2-dihydro-3*H*-1,2,4-triazole-3-thione,
2-[(3-Chlorophenyl)methyl]-5-(pyrimidin-5-yl)-1,2-dihydro-3*H*-1,2,4-triazole-3-thione,
5-(2-Chlorophenyl)-1,2-dihydro-2-[(3-cyanophenyl)methyl]-3*H*-1,2,4-triazole-3-thione,
5-(2-Chlorophenyl)-1,2-dihydro-2-[(4-nitrophenyl)methyl]-3*H*-1,2,4-triazole-3-thione,
5-(2-Chlorophenyl)-1,2-dihydro-2-(2-phenyl)ethyl-3*H*-1,2,4-triazole-3-thione,
5-(2-Chlorophenyl)-2-(2-(3-chlorophenyl)ethyl)-1,2-dihydro-3*H*-1,2,4-triazole-3-thione,
5-(2-Chlorophenyl)-1,2-dihydro-2-(3-phenyl)propyl-3*H*-1,2,4-triazole-3-thione,
and their pharmaceutically acceptable salts and solvates.

The present invention also provides a process for preparing a compound of formula (I) which comprises:
(a) reacting a compound of general formula (II), R¹-C(O)L, wherein L represents a leaving group (such as a halogen atom, e.g. chlorine) and R¹ is as defined in formula (I), with a compound of general formula 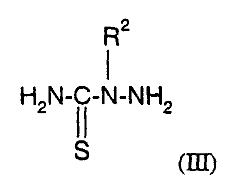 wherein R² is as defined in formula (I), followed by cyclisation; or
(b) reacting a compound of general formula 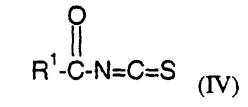 wherein R¹ is as defined in formula (I), with a compound of general formula

   R²-NHNH₂ (V)

   wherein R² is as defined in formula (I), followed by cyclisation; or
(c) reacting a compound of general formula 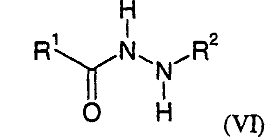 wherein R¹ and R² are as defined in formula (I), with ammonium thiocyanate, followed by cyclisation; or
(d) reacting a compound of general formula 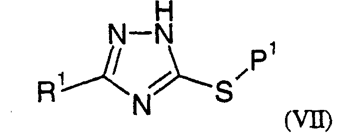
wherein P¹ represents a protecting group (e.g. methoxyethoxymethyl, triphenylmethyl or ethoxycarbonylethyl) and R¹ is as defined in formula (I), with a compound of general formula (VIII), R² - L¹, wherein L¹ represents a leaving group (e.g. a halogen atom such as bromine, or an alcoholic group under Mitsunobu conditions) and R² is as defined in formula (I), followed by removal of the protecting group P¹ (e.g. by using suitable acidic or basic conditions); and optionally after (a), (b), (c) or (d) forming a pharmaceutically acceptable salt or solvate of the compound of formula (I).

The process of the invention is conveniently carried out in an organic solvent such as dichloromethane, toluene, xylenes, triethylamine, pyridine or tetrahydrofuran at a temperature in the range, e.g. from 10 to 110°C. The cyclisation reaction may be effected under reflux conditions in the presence of sodium hydrogen carbonate, or sodium ethoxide in ethanol as described by H. Behringer *et al., Liebigs Ann. Chem.,* 1975, 1264-1271.

Compounds of formula (II), (III), (IV), (V), (VI) and (VIII) are either commercially available, are well known in the literature or may be prepared easily using known techniques.

Compounds of formula (VII) may be prepared by a process analogous to that of step (a) above using the compound 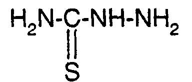 in place of the compound of formula (III).

It will be appreciated by those skilled in the art that in the processes described above the functional groups (e.g. hydroxyl groups) of intermediate compounds may need to be protected by protecting groups. The final stage in the preparation of the compounds of the invention may involve the removal of one or more protecting groups. The protection and deprotection of functional groups is fully described in 'Protective Groups in Organic Chemistry', edited by J. W. F. McOmie, Plenum Press (1973), and 'Protective Groups in Organic Synthesis', 2nd edition, T. W. Greene & P. G. M. Wuts, Wiley-Interscience (1991).

The compounds of formula (I) above may be converted to a pharmaceutically acceptable salt or solvate thereof, preferably an acid addition salt such as a hydrochloride, hydrobromide, phosphate, acetate, fumarate, maleate, tartrate, citrate, oxalate, methanesulphonate or *p*-toluenesulphonate, or an ammonium salt or an alkali metal salt such as a sodium or potassium salt.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention, particularly tautomers of general formula 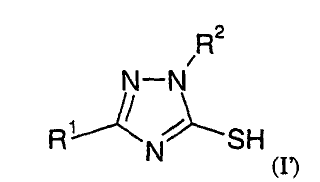 or 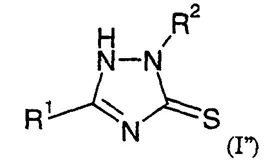 wherein R¹ and R² are as defined in formula (I) above.

The compounds of formula (I) have activity as pharmaceuticals, in particular as modulators of chemokine receptor (especially CXCR2) activity, and may be used in the treatment (therapeutic or prophylactic) of conditions/diseases in human and non-human animals which are exacerbated or caused by excessive or unregulated production of chemokines. Examples of such conditions/diseases include:
(1) **(the respiratory tract)** obstructive airways diseases including chronic obstructive pulmonary disease (COPD); asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) **(bone and joints)** rheumatoid arthritis, seronegative spondyloarthropathies (including ankylosing spondylitis, psoriatic arthritis and Reiter's disease), Behcet's disease, Sjogren's syndrome and systemic sclerosis;
(3) **(skin)** psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) **(gastrointestinal tract)** Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) **(other tissues and systemic disease)** multiple sclerosis, atherosclerosis, Acquired Immunodeficiency Syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura;
(6) **(allograft rejection)** acute and chronic following, for example, transplantation of kidney, heart, liver, lung, bone marrow, skin and cornea; and chronic graft versus host disease;
(7) cancers, especially non-small cell lung cancer (NSCLC) and squamous sarcoma;
(8) diseases in which angiogenesis is associated with raised CXCR2 chemokine levels (e.g. NSCLC); and
(9) cystic fibrosis, stroke, re-perfusion injury in the heart, brain, peripheral limbs and sepsis.

Thus, the present invention provides a compound of formula (I), or a pharmaceutically-acceptable salt or solvate thereof, as hereinbefore defined for use in therapy.

In a further aspect, the present invention provides the use of a compound of formula (I), or a pharmaceutically acceptable salt or solvate thereof, as hereinbefore defined in the manufacture of a medicament for use in therapy.

In a still further aspect, the present invention provides the use of a compound of formula (I), or a pharmaceutically acceptable salt or solvate thereof, as hereinbefore defined in the manufacture of a medicament for the treatment of human diseases or conditions in which modulation of chemokine receptor activity is beneficial.

In the context of the present specification, the term "therapy" also includes "prophylaxis" unless there are specific indications to the contrary. The terms "therapeutic" and "therapeutically" should be construed accordingly.

For the above-mentioned therapeutic uses the dosage administered will, of course, vary with the compound employed, the mode of administration, the treatment desired and the disorder indicated. For the treatment of an inflammatory disease, the daily dosage of the compound of formula (I) will typically be in the range from 0.001 mg/kg to 30 mg/kg.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally.

The invention will now be further illustrated by reference to the following examples. In the examples the Nuclear Magnetic Resonance (NMR) spectra were measured on a Varian Unity Inova 300 or 400 MHz spectrometer and the Mass Spectrometry (MS) spectra measured on a Finnigan Mat SSQ7000 or Micromass Platform spectrometer. Where necessary, the reactions were performed under an inert atmosphere of either nitrogen or argon. Chromatography was generally performed using Matrex Silica 60® (35-70 micron) or Prolabo Silica gel 60® (35-70 micron) suitable for flash silica gel chromatography.
High pressure liquid chromatography purification was performed using either a Waters Micromass LCZ with a Waters 600 pump controller, Waters 2487 detector and Gilson FC024 fraction collector or a Waters Delta Prep 4000. The abbreviations m.p. and DMSO used in the examples stand for melting point and dimethyl sulphoxide respectively.

### Example 1

### 5-(2,5-Dichlorophenyl)-1,2-dihydro-2-(phenylmethyl)-3H-1,2,4-triazole-3-thione

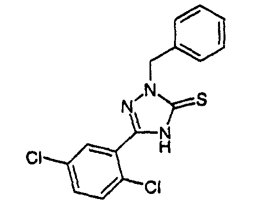

2,5-Dichlorobenzoic acid (0.11g), oxalyl chloride (0.1ml) and *N,N*-dimethylformamide (catalytic) in dichloromethane (1.7ml) was stirred at room temperature for 2 hours. The solution was evaporated and the residue was dissolved in pyridine (1.5ml) and 2-phenylmethyl thiosemicarbazide (*J. Chem. Soc.,* 1950, 1582) (0.1g) added. The solution was stirred at room temperature for 16 hours and then evaporated to a gum. 1M sodium hydrogen carbonate(1.5ml) was added and the mixture was heated at 100°C for 16 hours. The mixture was acidified with concentrated hydrochloric acid and the mixture extracted with ethyl acetate. The ethyl acetate solution was dried over magnesium sulphate and evaporated to dryness. Purification was chromatography eluting with 10%-100% ethyl acetate in isohexanes. The product was crystallised from diethyl ether. Yield 0.04g.
m.p.: 154-155°C
MS: APCI(+ve):336 (M+1,100%)
¹H NMR: δ (DMSO) 14.1 (br s, 1H), 7.8-7.9 (m, 1H), 7.6-7.7 (m, 3H), 7.3-7.4 (m, 4H), 5.4 (s, 2H)

### Example 2

### 2-[1,2-Dihydro-1-(phenylmethyl)-5-thioxo-3H-1,2,4-triazol-3-yl]benzoic acid, methyl ester

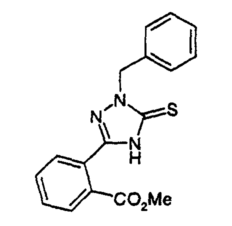

Methyl hydrogen phthalate (0.1g), oxalyl chloride (0.1ml) and *N,N*-dimethylformamide (catalytic) in dichloromethane (1.7ml) was stirred at room temperature for 2 hours. The solution was evaporated and the residue was dissolved in pyridine (1.5ml) and 2-phenylmethyl thiosemicarbazide (0.1g) added. The solution was stirred at room temperature for 16 hours and then evaporated to a gum. 1M sodium hydrogen carbonate(1.5ml) was added and the mixture was heated at 100°C for 16 hours. The mixture was acidified with concentrated hydrochloric acid and the precipitate collected. The crude carboxylic acid was dissolved in methanol (20ml) and trimethylsilylchloride (7ml) and stirred at room temperature for 16 hours and the solution was evaporated to dryness. Purification was by chromatography eluting with 30% ethyl acetate in isohexanes. Yield 0.013g.
m.p.: 129-130°C
MS: APCI(+ve):324 (M-1,100%)
¹H NMR: δ (DMSO) 13.9 (br s, 1H), 7.85-7.9 (m, 1H), 7.65-7.8 (m,3H), 7.3-7.4 (m, 5H), 5.35 (s, 2H), 3.6 (s,3H)

### Example 3

### 4-[1,2-Dihydro-1-(phenylmethyl)-5-thioxo-3H-1,2,4-triazol-3-yl]benzoic acid, methyl ester

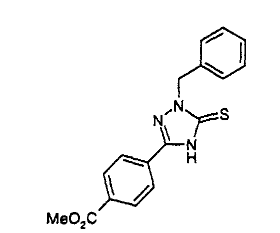

The title compound was prepared from mono-methyl terephthalate (0.1g) using the method of Example 2. Yield 0.013g.
m.p.: >215°C
MS: APCI(+ve):324 (M-1,100%)
¹H NMR: δ (DMSO) 14.3 (br s, 1H), 8.0-8.1 (m, 4H), 7.3-7.4 (m, 5H), 5.4 (s, 2H), 3.9 (s,3H)

### Example 4

### 5-(5-Chloro-2-thienyl)-1,2-dihydro-2-(phenylmethyl)-3H-1,2,4-triazole-3-thione

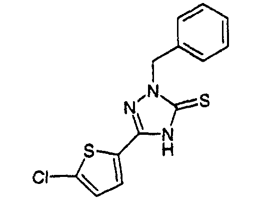

The title compound was prepared from 5-chlorothiophene-2-carboxylic acid (0.09g) using the method of Example 1. After chromatography the product was recrystallised from acetonitrile. Yield 0.013g.
m.p.: 79-80°C
MS: APCI(+ve):308 (M+1,100%)
¹H NMR: δ (DMSO) 14.3 (br s, 1H), 7.6 (d, 1H), 7.3-7.4 (m,5H), 7.3 (d, 1H), 5.3 (s, 2H)

### Example 5

### 5-(3-Chlorophenyl)-1,2-dihydro-2-(phenylmethyl)-3H-1,2,4-triazole-3-thione

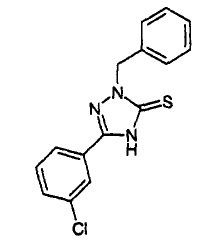

3-Chlorobenzoyl chloride (0.1g) was dissolved in pyridine (1.5ml) and 2-phenylmethyl thiosemicarbazide (0.1g) was added. The solution was stirred at room temperature for 16 hours and then evaporated to a gum. 1M sodium hydrogen carbonate(1.5ml) was added and the mixture was heated at 100°C for 16 hours. The mixture was acidified with concentrated hydrochloric acid and the mixture extracted with ethyl acetate. The ethyl acetate solution was dried over magnesium sulphate and evaporated to dryness. Purification was chromatography eluting with 10%-100% ethyl acetate in isohexanes. The product was crystallised from diethyl ether. Yield 0.1g.
m.p.: 175-176°C
MS: APCI(+ve):302 (M+1,100%)
¹H NMR: δ (DMSO) 14.2 (br s, 1H), 7.9-8.0 (m, 2H), 7.6 (m,2H), 7.4 (m, 5H), 5.4 (s, 2H)

### Example 6

### 1,2-Dihydro-5-(2-nitrophenyl)-2-(phenylmethyl)-3H-1,2,4-triazole-3-thione

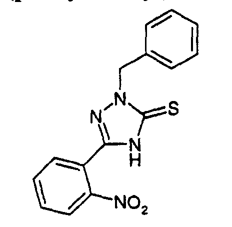

The title compound was prepared from 2-nitrobenzoyl chloride(0.1g) using the method of Example 5. Yield 0.02g.
m.p.: 147-148°C
MS: APCI(+ve):313 (M+1,100%)
¹H NMR: δ (DMSO) 14.15 (br s, 1H), 8.20 (m, 1H), 7.87 (m, 3H), 7.37 (m, 5H), 5.36 (s, 2H)

### Example 7

### 5-(4-Cyanophenyl)-1,2-dihydro-2-(phenylmethyl)-3H-1,2,4-triazole-3-thione

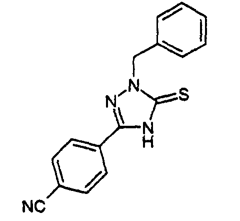

The title compound was prepared from 4-cyanobenzoyl chloride(0.1g) using the method of Example 5. Yield 0.02g.
m.p.: 237-238°C
MS: APCI(+ve):293 (M+1,100%)
¹H NMR: δ (DMSO) 14.39 (br s, 1H), 8.10 (d, 2H), 8.01 (d, 2H), 7.38 (m, 5H), 5.39 (s, 2H)

### Example 8

### 5-(2,3-Dichlorophenyl)-1,2-dihydro-2-(phenylmethyl)-3H-1,2,4-triazole-3-thione

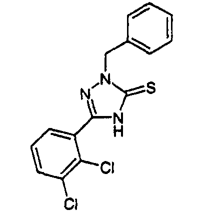

The title compound was prepared from 2,3-dichlorobenzoyl chloride(0.1g) using the method of Example 5. Yield 0.04g.
m.p.: 179-180°C
MS: APCI(+ve):336 (M+1,100%)
¹H NMR: δ (DMSO) 14.09 (br s, 1H), 7.88 (m, 1H), 7.68 (m, 1H), 7.55 (t, 1H), 7.38 (m, 5H), 5.39 (s, 2H)

### Example 9

### 5-(2-Bromophenyl)-1,2-dihydro-2-(phenylmethyl)-3H-1,2,4-triazole-3-thione

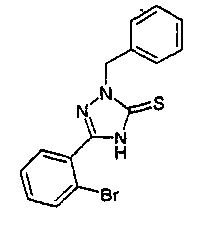

The title compound was prepared from 2-bromobenzoyl chloride (0.1g) using the method of Example 5. Yield 0.075g.
m.p.: 139-140°C
MS: APCI(+ve):346,348 (M+1,100%)
¹H NMR: δ (DMSO) 13.97 (br s, 1H), 7.80 (m, 1H), 7.62 (m, 1H), 7.51 (m, 2H),
7.35 (m, 5H), 5.38 (s, 2H)

### Example 10

### 1,2-Dihydro-2-(phenylmethyl)-5-[3-(trifluoromethyl)phenyl]-3H-1,2,4-triazole-3-thione

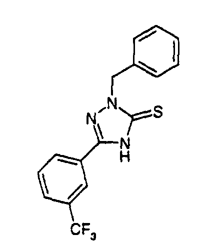

The title compound was prepared from 3-(trifluoromethyl)benzoyl chloride(0.1g) using the method of Example 5. Yield 0.05g.
m.p.: 209-210°C
MS: APCI(+ve):334 (M-1,100%)
¹H NMR: δ (DMSO) 14.33 (br s, 1H), 8.27 (br s, 1H), 8.19 (d, 1H), 7.9 (d, 1H), 7.77 (t, 1H), 7.3-7.4 (m, 5H), 5.4 (s, 2H)

### Example 11

### 5-(2-Furanyl)-1,2-dihvdro-2-(phenylmethyl)-3H-1,2,4-triazole-3-thione

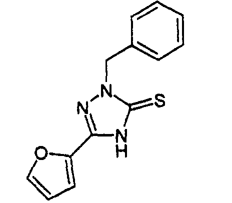

The title compound was prepared from 2-furoyl chloride (0.1g) using the method of Example 5. Yield 0.023g.
m.p.: 212-213°C
MS: APCI(+ve):258(M+1,100%)
¹H NMR: δ (DMSO) 14.17 (br s, 1H), 7.91 (s, 1H), 7.3-7.4 (m, 5H), 7.16 (d, 1H), 6.71 (m, 1H), 5.34 (s, 2H)

### Example 12

### 5-(2-Bromo-5-methoxyphenyl)-1,2-dihydro-2-(phenylmethyl)-3H-1,2,4-triazole-3-thione

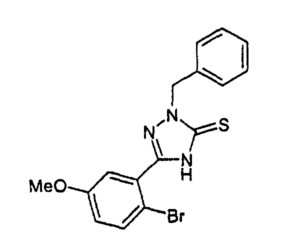

The title compound was prepared from 2-bromo-5-methoxybenzoyl chloride(0.1g) using the method of Example 5. Yield 0.026g.
m.p.: 154-155°C
MS: APCI(+ve): 376,378 (M+1,100%)
¹H NMR: δ (DMSO) 13.97 (br s, 1H), 7.67 (d, 1H), 7.3-7.4 (m, 5H), 7.24 (d, 1H), 7.07 (dd, 1H), 5.37 (s, 2H), 3.8 (s, 3H)

### Example 13

### 5-(2,5-Dichloro-3-thienyl)-1,2-dihydro-2-(phenylmethyl)-3H-1,2,4-triazole-3-thione

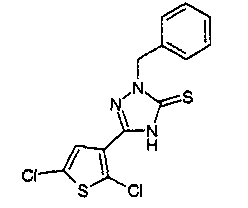

The title compound was prepared from 2,5-dichlorothiophene-3-carbonylchloride(0.1g) using the method of Example 5. Yield 0.019g.
m.p.: 160-162°C
MS: APCI(+ve):342 (M+1,100%)
¹H NMR: δ (DMSO) 14.04 (br s, 1H), 7.53 (s, 1H), 7.37 (m, 5H), 5.36 (s, 2H)

### Example 14

### 1,2-Dihydro-2-(phenylmethyl)-5-(4-pyridinyl)-3H-1,2,4-triazole-3-thione

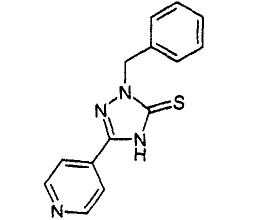

The title compound was prepared from isonicotinyl chloride hydrochloride(0. 1g) using the method of Example 5. Yield 0.05g.
m.p.: 160-162°C
MS: APCI(+ve):269 (M+1,100%)
¹H NMR: δ (DMSO) 14.1 (br s, 1H), 8.84 (m, 1H), 8.80 (m, 1H), 8.04 (m, 2H), 7.38 (m, 5H), 5.42 (s, 2H)

### Example 15

### 1,2-Dihydro-2-(phenylmethyl)-5-(4-phenylphenyl)-3H-1,2,4-triazole-3-thione

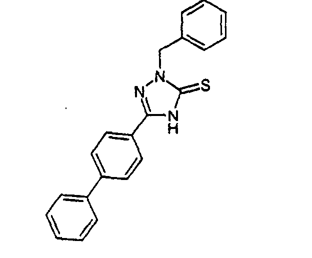

The title compound was prepared from 4-biphenylcarbonyl chloride(0.1g) using the method of Example 5. Yield 0.085g.
m.p.: 249-250°C
MS: APCI(+ve):344 (M+1,100%)
¹H NMR: δ (DMSO) 14.18 (br s, 1H), 8.01 (d, 2H), 7.82 (d, 2H), 7.78 (d, 2H), 7.51 (t, 2H), 7.41 (m, 6H), 5.40 (s, 2H)

### Example 16

### 5-(2-Chloro-3-pyridinyl)-1,2-dihydro-2-(phenylmethyl)-3H-1,2,4-triazole-3-thione

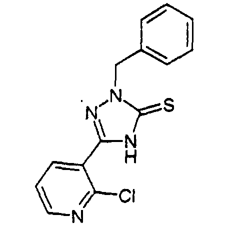

The title compound was prepared from 2-chloronicotinyl chloride(0.1g) using the method of Example 5. Yield 0.03g.
m.p.: 155-156°C
MS: APCI(+ve):303 (M+1,100%)
¹H NMR: δ (DMSO) 14.14 (br s, 1H), 8.61 (dd, 1H), 8.20 (dd, 1H), 7.61 (dd, 1H), 7.37 (m, 5H), 5.39 (s, 2H)

### Example 17

### 5-(2-Fluorophenyl)-1,2-dihydro-2-(phenylmethyl)-3H-1,2,4-triazole-3-thione

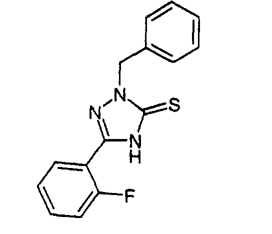

The title compound was prepared from 2-fluorobenzoyl chloride(0.09g) using the method of Example 5. Yield 0.047g.
m.p.: 180-183°C
MS: APCI(+ve):286 (M+1,100%)
¹H NMR: δ (DMSO) 14.03 (br s, 1H), 7.8-7.9 (m, 1H), 7.55-7.65 (m, 1H), 7.2-7.4 (m ,6H), 6.4 (m, 1H), 5.39 (s, 2H)

### Example 18

### 1,2-Dihydro-5-(2-methoxyphenyl)-2-(phenylmethyl)-3H-1,2,4-triazole-3-thione

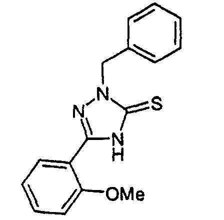

The title compound was prepared from 2-methoxybenzoyl chloride(0.1g) using the method of Example 5. Yield 0.065g.
m.p.: 142-143.5°C
MS: APCI(+ve):298 (M+1,100%)
¹H NMR: δ (DMSO) 13.40 (br s, 1H), 7.62 (dd, 1H), 7.51 (dt, 1H), 7.32 (m, 5H), 7.18 (d, 1H), 7.04 (t, 1H), 5.37 (s, 2H), 3.85 (s, 3H)

### Example 19

### 1,2-Dihydro-5-(2-methylphenyl)-2-(phenylmethyl)-3H-1,2,4-triazole-3-thione

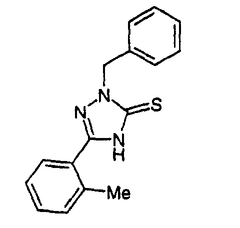

The title compound was prepared from 2-methylbenzoyl chloride(0.09g) using the method of Example 5. Yield 0.076g.
m.p.: 150-153°C
MS: APCI(+ve):282 (M+1,100%)
¹H NMR: δ (DMSO) 13.86 (br s, 1H), 7.61 (d, 1H), 7.36 (m, 8H), 5.38 (s, 2H), 2.43 (s, 3H)

### Example 20

### 5-(3-Chloro-2-thienvl)-1,2-dihvdro-2-(phenylmethyl)-3H-1,2,4-triazole-3-thione

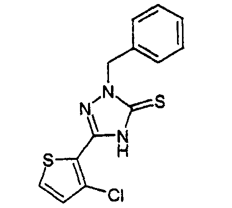

The title compound was prepared from 3-chlorothiophene-2-carboxylic acid(0.1g) using the method of Example 1. Yield 0.062g.
m.p.: 134-135°C
MS: APCI(+ve):308 (M+1)
¹H NMR: δ (DMSO) 14.12 (br s, 1H), 7.32 (m, 5H), 7.25 (d, 1H), 7.22 (d, 1H), 5.37 (s, 2H)

### Example 21

### 1,2-Dihydro-2-(phenylmethyl)-5-[4-(trifluoromethyl)phenyl]-3H-1,2,4-triazole-3-thione

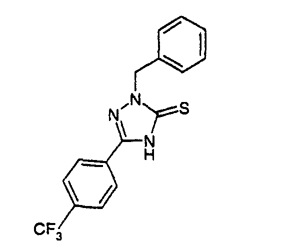

The title compound was prepared from 4-(trifluoromethyl)benzoic acid (0.1g) using the method of Example 1. Yield 0.047g.
m.p.: 201-203°C
MS: APCI(+ve): 336 (M+1)
¹H NMR: δ (DMSO) 14.35 (br s, 1H), 8.11 (d, 2H), 7.9 (d, 2H), 7.34 (m, 5H), 5.4 (s, 2H)

### Example 22

### 5-(2-Chloro-4-fluorophenyl)-1,2-dihydro-2-(phenylmethyl)-3H-1,2,4-triazole-3-thione

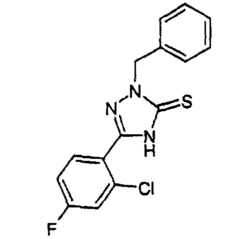

The title compound was prepared from 2-chloro-4-fluorobenzoic acid (0.1g) using the method of Example 1. Yield 0.029g.
m.p.: 135-136°C
MS: APCI(+ve): 320(M+1)
¹H NMR: δ (DMSO) 14.02 (br s, 1H), 7.7-7.9 (m, 2H), 7.3-7.5 (m, 6H), 5.38 (s, 2H)

### Example 23

### 1,2-Dihydro-5-(4-methoxyphenyl)-2-(phenylmethyl)-3H-1,2,4-triazole-3-thione

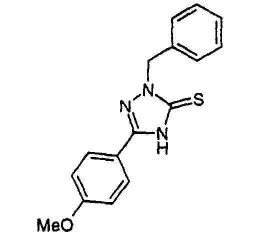

4-Methoxybenzoyl isothiocyanate (0.38g), benzylhydrazine (0.38g) and triethylamine (0.53ml) in toluene (10ml) was heated at 80°C. After 1 hour the solution was evaporated under reduced pressure. The residue was suspended in 1M sodium hydrogen carbonate (4ml) and heated under reflux for 16 hours. The mixture was acidified with 2N hydrochloric acid and the resulting solid was collected and recrystallised from ethanol. Yield 0.087g.
m.p.: 235-237°C
MS: APCI(+ve):298 (M+1,100%)
¹H NMR: δ (DMSO) 13.97 (br s, 1H), 7.85 (d, 2H), 7.28-7.39 (m, 5H), 7.07 (d, 2H), 5.36 (s, 2H), 3.81 (s, 3H)

### Example 24

### 1,2-Dihydro-5-(4-methylphenyl)-2-(phenylmethyl)-3H-1,2,4-triazole-3-thione

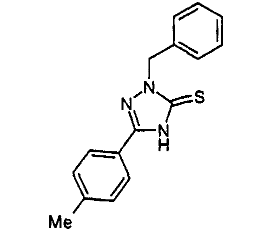

The title compound was prepared from 4-methylbenzoyl chloride (0.187g) using the method of Example 5. After acidification, the precipitate was collected and recrystallised from acetonitrile. Yield 0.14g.
m.p.: 234-235°C
MS: APCI(+ve):282 (M+1,100%)
¹H NMR: δ (DMSO) 14.04 (br s, 1H), 7.79 (d, 2H), 7.35-7.4(m, 7H), 5.36 (s, 2H), 2.35 (s, 3H)

### Example 25

### 5-(3,4-Dichlorophenyl)-1,2-dihydro-2-(phenylmethyl)-3H-1,2,4-triazole-3-thione

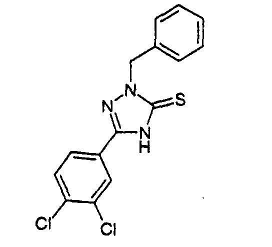

The title compound was prepared from 3,4-dichlorobenzoyl chloride(0.25g) using the method of Example 24. Yield 0.11g.
m.p.: 227-228°C
MS: APCI(+ve):336 (M+1,100%)
¹H NMR: δ (DMSO) 14.25 (br s, 1H), 8.14 (s, 1H), 7.8 (m, 2H), 7.35-7.4 (m, 5H), 5.37(s, 2H)

### Example 26

### 5-(4-Bromophenyl)-1,2-dihydro-2-(phenylmethyl)-3H-1,2,4-triazole-3-thione

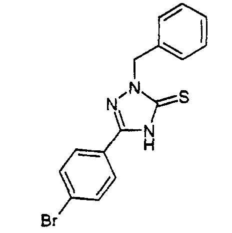

The title compound was prepared from 4-bromobenzoyl chloride(0.266g) using the method of Example 24. Yield 0.06g.
m.p.: 250-251°C
MS: APCI(+ve):346, 348 (M+1,100%)
¹H NMR: δ (DMSO) 14.20 (br s, 1H), 7.83(d, 2H), 7.35(d, 2H), 7.25-7.40 (m, 5H), 5.37 (s, 2H)

### Example 27

### 5-(2,4-Dichlorophenyl)-1,2-dihydro-2-(phenylmethyl)-3H-1,2,4-triazole-3-thione

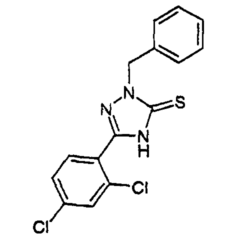

The title compound was prepared from 2,4-dichlorobenzoyl chloride(0.23g) using the method of Example 5. After acidification the precipitate was collected and recrystallised from ethanol. Yield 0.055g.
m.p.: 169-170°C
MS: APCI(+ve):336, 338 (M+1)
¹H NMR: δ (DMSO) 14.07 (br s, 1H), 7.86 (d, 1H), 7.7 (d, 1H), 7.6 (dd, 1H), 7.3-7.4 (m, 5H), 5.38 (s, 2H)

### Example 28

### 5-(2-Chlorophenyl)-1,2-dihydro-2-(phenylmethyl)-3H-1,2,4-triazole-3-thione

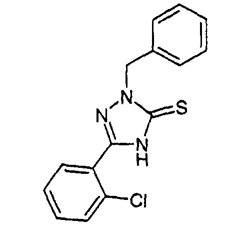

N,N'-Carbonyldiimidazole (0.21g) was added to 2-chlorobenzoic acid ( 0.2g) in tetrahydrofuran (2.5ml). After 1 hour benzylhydrazine ( 0.25g) and sodium hydride (0.039g, 60% dispersion in oil) in N,N-dimethylformamide (1.25ml) was added. After 48 hours the solvent was evaporated under reduced pressure. The residue was dissolved in ethyl acetate, washed with water, dried over magnesium sulphate and evaporated. The resulting gum was dissolved in ethanol (10ml). 1M hydrochloric acid in diethyl ether (1.2ml) was added and the mixture was evaporated. The residue and ammonium thiocyanate (0.088g) in ethanol (10ml) was heated under reflux for 18 hours. The solution was evaporated under reduced pressure and 1M sodium hydrogen carbonate (2ml) was added. The mixture was heated at 100°C for 18 hours. The mixture was acidified with 2M hydrochloric acid, extracted with ethyl acetate, dried over magnesium sulphate and evaporated under reduced pressure. Purification was by chromatography eluting with 30% ethyl acetate in isohexanes. Yield 0.115g.
m.p.: 150-151°C
MS: APCI(+ve):302,304 (M+1)
¹H NMR: δ (DMSO) 14.0 (br s, 1H), 7.64 (dt, 2H), 7.51 (dt, 1H), 7.49 (dt, 1H), 7.35 (m, 5H), 5.38 (s, 2H)

### Example 29

### 5-(3,5-Dichlorophenyl)-1,2-dihydro-2-(phenylmethyl)-3H-1,2,4-triazole-3-thione

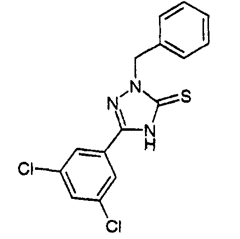

The title compound was prepared from 3,5-dichlorobenzoyl chloride(0.12g) using the method of Example 5. Yield 0.097g.
m.p.: 205-212°C
MS: APCI(+ve):336 (M+1)
¹H NMR: δ (DMSO) 14.15 (br s, 1H), 7.92 (d, 2H), 7.8 (t, 1H), 7.35 (m, 5H), 5.38 (s, 2H)

### Example 30

### 1,2-Dihydro-5-(3-phenoxyphenyl)-2-(phenylmethyl)-3H-1,2,4-triazole-3-thione

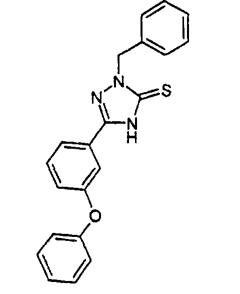

The title compound was prepared from 3-phenoxybenzoic acid(0.12g) using the method of Example 1. Yield 0.068g.
m.p.: 200-202°C
MS: APCI(+ve):360 (M+1,100%)
¹H NMR: δ (DMSO) 14.16 (br s, 1H), 7.67 (d, 1H), 7.52 (m, 2H), 7.36 (m, 7H), 7.18 (m, 2H), 7.07 (d, 2H), 5.36 (s, 2H)

### Example 31

### 1,2-Dihydro-2-(phenylmethyl)-5-(2-thienyl)-3H-1,2,4-triazole-3-thione

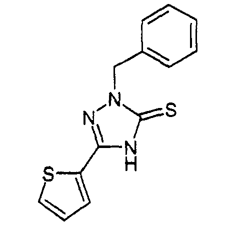

The title compound was prepared from thiophene-2-carbonylchloride(0.3g) using the method of Example 24. Yield 0.25g.
m.p.: 230-231°C
MS: APCI(+ve):274 (M+1,100%)
¹H NMR: δ (DMSO) 14.26 (br s, 1H), 7.99 (m, 2H), 7.38 (m, 5H), 7.2 (m, 1H), 5.34 (s, 2H)

### Example 32

### 2-[(3-Chlorophenyl)methyl]-5-(2,4-dichlorophenyl)-1,2-dihydro-3H-1,2,4-triazole-3- thione

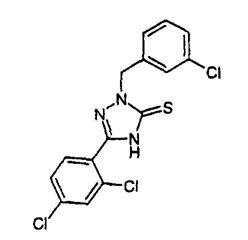

### i) 2,4-Dichlorobenzoic acid [(3-chlorophenyl)methylene]hydrazide

A solution of 3-chlorobenzaldehyde (0.28g) and 2,4-dichlorobenzhydrazide (0.4g) in methanol (14ml) was treated with concentrated hydrochloric acid (0.1ml). After 16 hours the precipitate was collected and washed with diethyl ether. Used directly.

### ii) 2,4-Dichlorobenzoic acid 2-[(3-chlorophenyl)methyl]hydrazide

Triethylsilane (0.47g) was added to a stirred solution of the product of step (i) in trifluoroacetic acid (3ml) cooled at 0°C. After 4 hours the solution was allowed to warm to room temperature overnight. The solution was evaporated under reduced pressure. The residue was suspended in water and the pH adjusted to 11 with potassium hydroxide. The mixture was extracted with ethyl acetate. The organic solution was washed with brine, dried over magnesium sulphate and evaporated under reduced pressure. Used directly.

### iii) 2-[(3-Chlorophenyl)methyl]-5-(2,4-dichlorophenyl)-1,2-dihydro-3H-1,2,4-triazole-3-thione

The product of step (ii) was dissolved in methanol (10ml). 1M hydrochloric acid in diethyl ether (1.5ml) was added and the mixture was evaporated. The residue and ammonium thiocyanate (0.16g) in ethanol (4ml) was heated at 75°C for 18 hours. The solution was evaporated under reduced pressure and 1M sodium hydrogen carbonate (10ml) was added. The mixture was heated at 75°C for 18 hours. The mixture was acidified with 2M hydrochloric acid, extracted with ethyl acetate, dried over magnesium sulphate and evaporated under reduced pressure. Purification was by chromatography eluting with 30% ethyl acetate in isohexanes. Yield 0.011g.
m.p.: 190-191°C
MS: APCI(+ve): 370(M+1)
¹H NMR: δ (DMSO) 14.12 (br s, 1H), 7.87 (d, 1H), 7.72 (d, 1H), 7.62 (dd, 1H), 7.3-7.5 (m, 4H), 5.4 (s, 2H)

### Example 33

### 5-(2,4-Dichlorophenyl)-1,2-dihydro-2-[(3-methoxyphenyl)methyl]-3H-1,2,4-triazole-3- thione

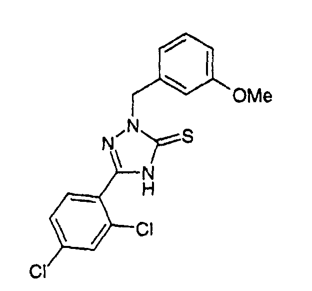

The title compound was prepared from 3-methoxybenzaldehyde(0.27g) using the method of Example 32. Yield 0.037g.
m.p.: 173-174°C
MS: APCI(+ve): 366(M+1)
¹H NMR: δ (DMSO) 14.07 (br s, 1H), 7.86 (d, 1H), 7.19 (d, 1H), 7.61 (dd, 1H), 7.28 (t, 1H), 6.91 (m, 3H), 5.34 (s, 2H), 3.73 (s, 3H)

### Example 34

### 5-(2,4-Dichlorophenyl)-1,2-dihydro-2-[(3-phenoxyphenyl)methyl]-3H-1,2,4-triazole-3- thione

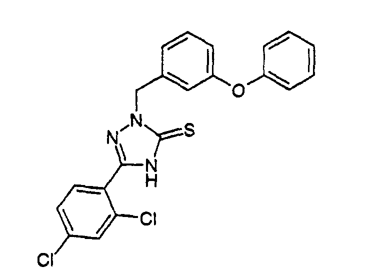

The title compound was prepared from 3-phenoxybenzaldehyde(0.4g) using the method of Example 32. Yield 0.18g.
m.p.: 180-181°C
MS: APCI(+ve): 428(M+1)
¹H NMR: δ (DMSO) 14.06 (br s, 1H), 7.86 (d, 1H), 7.68 (d, 1H), 7.62 (dd, 1H), 7.39 (m, 3H), 7.14 (m, 2H), 7.0 (m, 3H), 6.94 (dd, 1H), 5.37 (s, 2H)

### Example 35

### 1,2-Dihydro-2-[(3-hydroxyphenyl)methyl]-5-phenyl-3H-1,2,4-triazole-3-thione

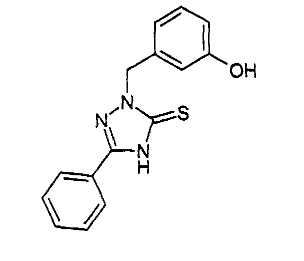

The title compound was prepared from benzoyl isothiocyanate(0.19ml) and 3-hydroxybenzylhydrazine hydrochloride (0.3g) using the method of Example 23. The product was recrystallised from acetonitrile/water. Yield 0.045g.
m.p.: 233-235°C
MS: APCI(+ve): 284(M+1,100%)
¹H NMR: δ (DMSO) 14.14 (br s, 1H), 9.45 (s, 1H), 7.91 (d, 2H), 7.54-7.49 (m, 3H), 7.14 (t, 1H), 6.79 (d, 1H), 6.74 (s, 1H), 6.67 (d, 1H), 5.28 (s, 2H)

### Example 36

### 5-(2-Chloro-6-fluorophenyl)-1,2-dihydro-2-(phenylmethyl)-3H-1,2,4-triazole-3-thione

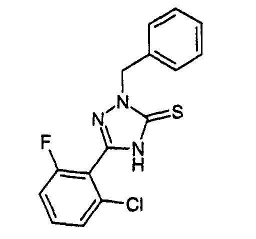

The title compound was prepared from 2-chloro-6-fluorobenzoic acid (0.09g) using the method of Example 1. After chromatography the product was crystallised from diethyl ether/isohexanes. Yield 0.0023g.
m.p.: 150-151°C
MS: APCI(+ve):320 (M+1,100%)
¹H NMR: δ (DMSO) 14.15 (br s, 1H), 7.65-7.8 (s, 1H), 7.6 (s, 1H), 7.5 (t, 1H),
7.3-7.4 (m,5H), 5.4 (s, 2H)

### Example 37

### 1,2-Dihydro-5-(1-naphthalenyl)-2-(phenylmethyl)-3H-1,2,4-triazole-3-thione

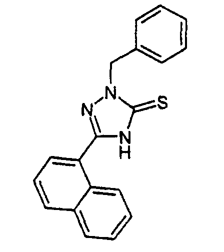

The title compound was prepared from 1-naphthoyl chloride(0.1 g) using the method of Example 5. Yield 0.002g.
MS: APCI(+ve):318 (M+1,100%)
¹H NMR: δ (DMSO) 14.2 (br s, 1H), 8.62 (m, 1H), 7.9-8.1 (m,3H), 7.62 (m, 3H), 7.38 (m, 5H), 5.47 (s, 2H)

### Example 38

### 1,2-Dihydro-5-[2-(methylthio)phenyl]-2-(phenylmethyl)-3H-1,2,4-triazole-3-thione

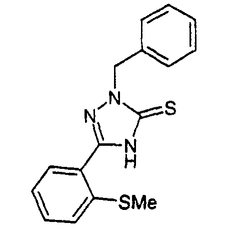

The title compound was prepared from 2-methylthiobenzoic acid(0.1g) using the method of Example 1. Yield 0.035g.
m.p.: 170-171°C
MS: APCI(+ve):314 (M+1,100%)
¹H NMR: δ (DMSO) 13.90 (br s, 1H), 7.61 (d, 1H), 7.51 (m,1H), 7.42 (d, 1H),
7.25-7.4 (m, 6H), 5.37 (s, 2H), 2.44 (s, 3H)

### Example 39

### 1,2-Dihydro-5-[2-(phenoxy)phenyl]-2-(phenylmethyl)-3H-1,2,4-triazole-3-thione

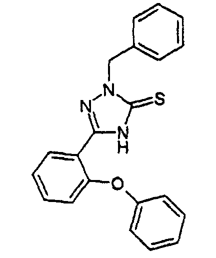

The title compound was prepared from 2-(phenoxy)benzoic acid(0.12g) using the method of Example 1. Yield 0.012g.
m.p.: 158-159°C
MS: APCI(+ve):360 (M+1)
¹H NMR: δ (DMSO) 13.83 (br s, 1H), 7.77 (d, 1H), 7.52 (t,1H), 7.2-7.4 (m, 8H), 6.9-7.05 (m, 4H), 5.39 (s, 2H)

### Example 40

### 1,2-Dihydro-5-(3-(phenylcarbonyl)phenyl)-2-(phenylmethyl)-3H-1,2,4-triazole-3- thione

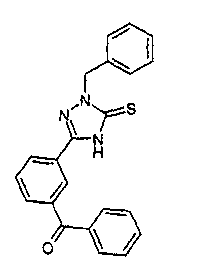

The title compound was prepared from 3-(phenylcarbonyl)benzoic acid(0.12g) using the method of Example 1. After chromatography the product was recrystallised from acetonitrile. Yield 0.035g.
m.p.: 154-155°C
MS: APCI(+ve):372 (M+1)
¹H NMR: δ (DMSO) 14.29 (br s, 1H), 8.25 (s, 1H), 8.20 (d,1H), 7.85 (d, 1H), 7.8 (m, 2H), 7.71 (m, 2H), 7.60 (m, 2H), 7.35 (m, 5H), 5.39 (s, 2H)

### Example 41

### 1,2-Dihydro-5-(1-bromo-2-naphthalenyl))-2-(phenylmethyl)-3H-1,2,4-triazole-3-thione

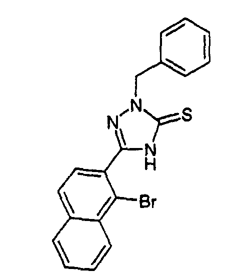

The title compound was prepared from 1-bromo-2-naphthoic acid(0.12g) using the method of Example 1. Yield 0.023g.
m.p.: 168-170°C
MS: APCI(+ve):396-398 (M+1)
¹H NMR: δ (DMSO) 14.01 (br s, 1H), 8.32 (d, 1H), 8.12 (m, 2H), 7.81 (m, 2H), 7.63 (d, 1H), 7.40 (m, 5H), 5.42 (s, 2H)

### Example 42

### 5-(2-Chlorophenyl)-2-[(3-chlorophenyl)methyl]-1,2-dihydro-3H-1,2,4-triazole-3-thione

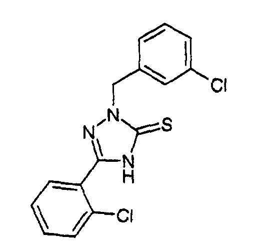

The title compound was prepared from 3-chlorobenzaldehyde(16.48g) and 2-chlorobenzhydrazide (20.0g) using the method of Example 32. Yield 8.67g.
m.p.: 143-143.5°C
MS: APCI(+ve):336 (M+1)
¹H NMR: δ (DMSO) 14.05 (br s, 1H), 7.69 (dd, 1H), 7.65 (dd, 1H), 7.59 (dt, 1H), 7.50 (dt, 1H), 7.3-7.45 (m, 4H), 5.40 (s, 2H)

### Example 43

### 5-(2,4-Dichlorophenyl)-1,2-dihydro-2-[(3-methylphenyl)methyl]-3H-1,2,4-triazole-3-thione

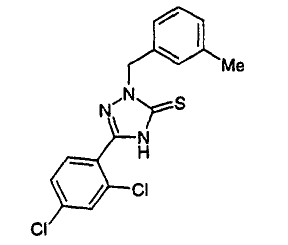

The title compound was prepared from 3-methylbenzaldehyde(0.24g) using the method of Example 32. Yield 0.18g.
m.p.: 177-178°C
MS: APCI(+ve):350 (M+1)
¹H NMR: δ (DMSO) 14.05 (br s, 1H), 7.86 (d, 1H), 7.32 (d, 1H), 7.59 (dd, 1H), 7.25 (t, 1H), 7.1-7.2 (m, 3H), 5.33 (s, 2H), 2.29 (s, 3H)

### Example 44

### 5-(2,4-Dichlorophenyl)-1,2-dihydro-2-(2-phenylethyl)-3H-1,2,4-triazole-3-thione

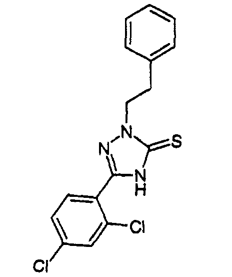

The title compound was prepared from phenylacetaldehyde(0.36g) using the method of Example 32. Yield 0.016g.
m.p.: 179-182°C
MS: APCI(+ve):352 (M+1)
¹H NMR: δ (DMSO) 13.92 (br s, 1H), 7.86 (s, 1H), 7.58-7.7 (m, 2H), 7.1-7.3 (m, 5H), 4.36 (t, 2H), 3.15 (t, 2H)

### Example 45

### 2-[(4-Chlorophenyl)methyl]-5-(2,4-dichlorophenyl)-1,2-dihydro-3H-1,2,4-triazole-3- thione

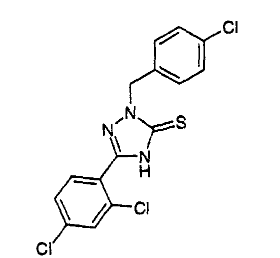

The title compound was prepared from 4-chlorobenzaldehyde(0.4g) using the method of Example 32. Yield 0.0.036g.
m.p.: 205-206°C
MS: APCI(+ve): 370 (M+1)
¹H NMR: δ (DMSO) 14.10 (br s, 1H), 7.86 (s, 1H), 7.72 (d, 1H), 7.61 (d, 1H), 7.35-7.45 (m, 4H), 5.38 (s, 2H)

### Example 46

### 5-(4-Bromophenyl)-2-[(3-chlorophenyl)methyl]-1,2-dihydro-3H-1,2,4-triazole-3- thione

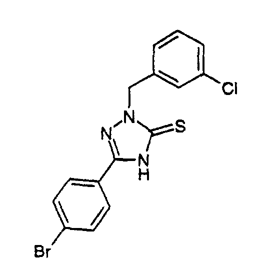

### i) 2-(3-Chlorophenyl)methyl thiosemicarbazide

(3-Chlorophenyl)methylhydrazine hydrochloride (50.0g) and ammonium thiocyanate (19.8g) were heated at reflux in ethanol (200ml) overnight. The hot suspension was filtered and the filtrate was allowed to cool and crystallize. The crystals were removed and dried to give the subtitle compound as colourless needles (27.6g).
m.p.: 153-158°C
MS: ESI(+ve) 216 (M+1, 100%)
¹H NMR: δ (DMSO) 7.65 (br, 2H), 7.42-7.27 (m, 4H), 5.22 (s, 2H), 4.75 (s, 2H)

### ii) 5-(4-Bromophenyl)-2-[(3-chlorophenyl)methyl]-1,2-dihydro-3H-1,2,4-triazole-3-thione

The title compound was prepared from 4-bromobenzoic acid (1.4g) and the product from step (i) (1.5g) using the method of Example 1. After acidification, the precipitate was collected by filtration. Yield 2.6g.
m.p.: 242-243°C
MS: ESI(-ve) 378/380 (M-1), 378 (100%)
¹H NMR: δ (DMSO) 14.23 (br, 1H), 7.85 (d, 2H), 7.74 (d, 2H), 7.45-7.32 (m, 4H), 5.39 (s, 2H)

### Example 47

### 2-[(3-Chlorophenyl)methyl]-5-(4-methylphenyl)-1,2-dihydro-3H-1,2,4-triazole-3- thione

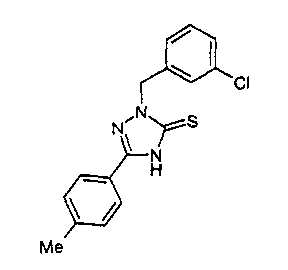

Prepared by the method of Example 46, step ii) using 4-methylbenzoic acid (0.13g). The crude product was purified by recrystallization from aqueous acetonitrile. Yield (0.16g).
m.p.: 207-208°C
MS: ESI(+ve) 316 (M+1, 100%)
¹H NMR: δ (DMSO) 14.08 (br, 1H), 7.80 (d, 2H), 7.44-7.31 (m, 6H), 5.38 (s, 2H), 2.36 (s, 3H)

### Example 48

### 2-[(3-Chlorophenyl)methyl]-5-(4-methoxyphenyl)-1,2-dihydro-3H-1,2,4-triazole-3- thione

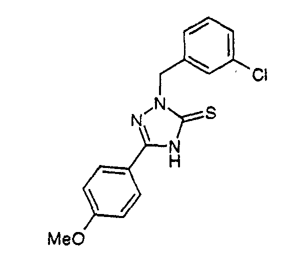

Prepared by the method of Example 46 step ii) using 4-methoxybenzoic acid (0.14g).
Yield (0.20g).
m.p.: 231-233°C
MS: ESI(+ve) 332 (M+1, 100%)
¹H NMR: δ (DMSO) 14.01 (br, 1H), 7.86 (d, 2H), 7.44-7.31 (m, 4H), 7.08 (d, 2H), 5.37 (s, 2H), 3.82 (s, 3H)

### Example 49

### 2-[(3-Chlorophenyl)methyl]-5-[4-(1-methylethyl)phenyl]-1,2-dihydro-3H-1,2,4-triazole-3-thione

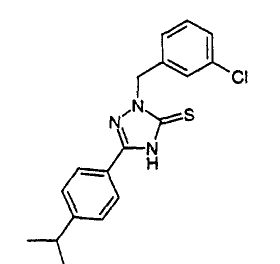

Prepared by the method of Example 46 using 4-(isopropyl)benzoic acid (0.15g). Yield (0.18g).
m.p.: 195-196°C
MS: ESI(+ve) 334 (M+1, 100%)
¹H NMR: δ (DMSO) 14.10 (br, 1H), 7.84 (d, 2H), 7.44-7.31 (m, 6H), 5.39 (s, 2H), 2.98-2.89 (m, 1H), 1.21 (d, 6H)

### Example 50

### 5-(2-Chlorophenyl)-1,2-dihydro-2-(4-methylphenyl)methyl-3H-1,2,4-triazole-3-thione

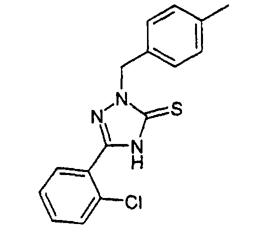

### (a) 5-(2-Chlorophenyl)-3-[(2-methoxy)ethyloxymethylthio]-1H-1,2,4-triazole

5-(2-Chlorophenyl)-1,2-dihydro-1*H*-1,2,4-triazole-3-thione (5.0g) and potassium carbonate (3.26g) were stirred in dry DMF (40ml). Methoxyethylmethyl chloride (2.7ml) was added and the mixture stirred 5 hrs. The mixture was poured onto water containing sodium chloride and extracted into ethyl acetate. The extracts were washed with saturated sodium chloride solution then dried over magnesium sulfate and evaporated under reduced pressure. Purification was by chromatography eluting with 10% ethyl acetate in dichloromethane to afford an oil. Yield 2.60g
MS: APCI(+ve): 300/302(M+1)
¹H NMR: δ (DMSO) 8.04 (m, 1H), 7.47 (m, 1H), 7.35 (m, 2H), 5.20 (s, 2H), 3.92 (m, 2H), 3.64 (m, 2H), 3.46 (s, 3H)

### (b)5-(2-Chlorophenyl)-1,2-dihydro-2-(4-methylphenyl)methyl-3H-1,2,4-triazole-3-thione

5-(2-Chlorophenyl)-3-[(2-methoxy)ethyloxymethylthio]-1*H*-1,2,4-triazole (0.21g) (product of step a)), potassium carbonate (0.097g) and 4-methylphenylmethyl bromide (0.13g) were stirred in dry DMF (1ml) for 16hrs. Conc. hydrochloric acid (0.5ml) was added and stirring continued 1hr. Water was added and the mixture extracted with ethyl acetate. The solution was dried over magnesium sulfate and evaporated under reduced pressure. The residue was dissolved in acetonitrile (2ml) and conc. hydrochloric acid (1ml) added. The solution was stood 16hrs and the solid which had separated was collected. Purification was by chromatography eluting with 5% ethyl acetate in dichloromethane. Yield 0.049g
m.p.: 162-163°C
MS: APCI(+ve): 316 (M+1)
¹H NMR: δ (DMSO) 13.97 (s, 1H), 7.66 (m, 2H), 7.58 (td, 1H), 7.49 (td, 1H),
7.27 (d, 2H), 7.17 (d, 2H), 5.32 (s, 2H), 2.28 (s, 3H)

### Example 51

### 5-(2-Chlorophenyl)-1,2-dihydro-2-[(4-(1,1-dimethylethyl)phenyl)methyl]-3H-1,2,4-triazole-3-thione

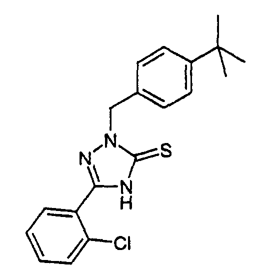

The title compound was prepared from 4-(1,1-dimethylethyl)phenylmethyl bromide (0.129ml) using the method of Example 50, step b). Yield 0.022g.
m.p.: 211-215°C
MS: APCI(+ve): 358 (M+1)
¹H NMR: δ (DMSO) 13.98 (s, 1H), 7.66 (m, 2H), 7.58 (t, 1H), 7.49 (t, 1H), 7.38 (d, 2H), 7.31 (d, 2H), 5.33 (s, 2H), 1.26 (s, 9H)

### Example 52

### 5-(2-Chlorophenyl)-1,2-dihydro-2-[(3-phenyloxy)phenylmethyl]-3H-1,2,4-triazole-3-thione

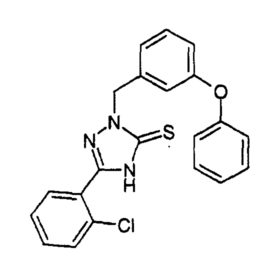

### (a) 5-(2-Chlorophenyl)-3-triphenylmethylthio-1H-1,2,4-triazole

5-(2-Chlorophenyl)-1,2-dihydro-1*H*-1,2,4-triazole-3-thione (1.0g) and potassium carbonate (0.7g) were stirred in dry DMF (40ml). Triphenylmethyl chloride (1.31g) was added and the mixture stirred 16 hrs. The mixture was poured slowly onto water and stirred 30mins. The solid which separated was collected, washed with water and dried. Yield 1.54g
MS: APCI(-ve):452/454(M-1)
¹H NMR: δ (DMSO) 14.23 (s, 1H), 7.30 (m, 19H)

### (b) 5-(2-Chlorophenyl)-1,2-dihydro-2-[(3-phenyloxy)phenylmethyl]-3H-1,2,4-triazole-3-thione

5-(2-Chlorophenyl)-3-triphenylmethylthio-1*H*-1,2,4-triazole (0.454g) (product of step a), potassium carbonate (140mg) and 3-phenyloxyphenylmethyl chloride (0.219g) were stirred together in dry DMF (1ml) for 8hrs. Conc. hydrochloric acid (0.5ml) and acetonitrile (0.5ml) were added and the mixture stirred 16hrs. Water was added and the mixture extracted with ethyl acetate. The extracts were washed with saturated sodium chloride solution. The solution was dried over magnesium sulfate and evaporated under reduced pressure. Purification was by chromatography eluting with 2% ethyl acetate in dichloromethane to give an oil. Trituration with ether/ isohexane gave a solid which was collected and dried. Yield 0.094g
m.p.: 137-138°C
MS: APCI(+ve): 394(M+1)
¹H NMR: δ (DMSO) 14.01 (s, 1H), 7.64 (m, 3H), 7.50 (t, 1H), 7.38 (m, 3H),
7.14 (m, 2H), 7.01 (m, 2H), 6.94 (d, 1H), 5.37 (s, 2H)

### Example 53

### 5-(2-Chlorophenyl)-1,2-dihydro-2-[[4-(trifluoromethyl)phenyl]methyl]-3H-1,2,4-triazole-3-thione

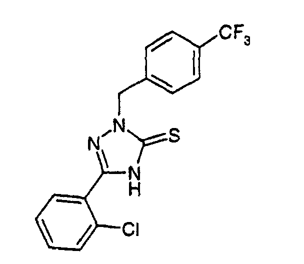

The title compound was prepared from 4-(trifluoromethyl)phenylmethyl bromide (0.167g) using the method of Example 50, step b). Yield 0.05g.
m.p.: 167-168°C
MS: APCI(+ve): 370(M+1)
¹H NMR: δ (DMSO) 14.09 (s, 1H), 7.76 (d, 2H), 7.64 (m, 3H), 7.58 (d, 2H), 7.50 (td, 1H), 5.50 (s, 2H)

### Example 54

### 5-(2-Chlorophenyl)-1,2-dihydro-2-[(4-(methoxyphenyl)methyl)-3H-1,2,4-triazole-3-thione

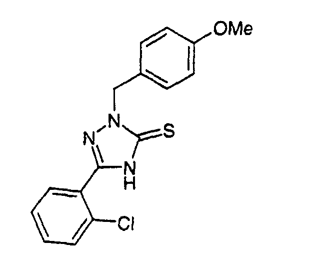

The title compound was prepared from 4-methoxyphenylmethyl bromide(0.167g) using the method of Example 52. Yield 0.028g.
m.p.: 153-154°C
MS: APCI(+ve): 332(M+1)
¹H NMR: δ (DMSO) 13.94 (s, 1H), 7.64 (m, 2H), 7.58 (t, 1H), 7.49 (t, 1H), 7.35 (d, 2H), 6.93 (s, 2H), 5.30 (s, 2H), 3.74 (s, 3H)

### Example 55

### 2-[(3-Chlorophenyl)methyl]-5-(4-phenoxyphenyl)-1,2-dihydro-3H-1,2,4-triazole-3- thione

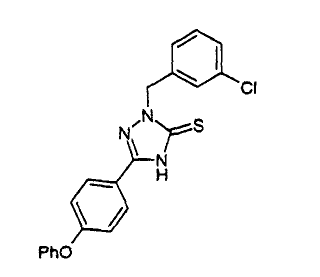

Prepared by the method of Example 46 using 4-phenoxybenzoic acid (0.20g). The crude product was purified by two recrystallizations, one from aqueous acetonitrile and one from ethyl acetate/isohexane. Yield (0.081g).
m.p.: 183-184°C
MS: ESI(+ve) 394 (M+1, 100%)
¹H NMR: δ (DMSO) 14.12 (br, 1H), 7.92 (d, 2H), 7.47-7.31 (m, 6H), 7.22 (t, 1H), 7.11-7.09 (m, 4H), 5.38 (s, 2H)

### Example 56

### 2-[(3-Chlorophenyl)methyl]-5-(3-pyridinyl)-1,2-dihydro-3H-1,2,4-triazole-3-thione

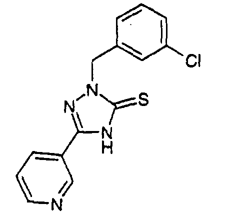

Prepared by the method of Example 46 using 3-pyridinecarboxylic acid (0.12g). The crude product was purified by recrystallization from aqueous methanol. Yield (0.094g).
m.p.: 208-209°C
MS: ESI(+ve) 303 (M+1, 100%)
'H NMR: δ (DMSO) 14.35 (br, 1H), 9.08-9.07 (m, 1H), 8.72-8.70 (m, 1H), 8.27-8.24 (m, 1H), 7.58-7.55 (m, 1H), 7.46-7.33 (m, 4H), 5.42 (s, 2H)

### Example 57

### 2-[(3-Chlorophenyl)methyl]-5-phenyl-1,2-dihydro-3H-1,2,4-triazole-3-thione

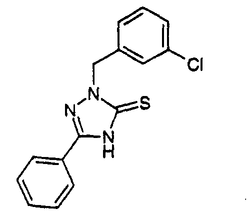

Prepared by the method of Example 46 using benzoic acid (0.11g). The crude product was purified by recrystallization from aqueous acetonitrile. Yield (0.14g).
m.p.: 213°C
MS: ESI(+ve) 302 (M+1, 100%)
¹H NMR: δ (DMSO) 14.18 (br, 1H), 7.94-7.89 (m, 2H), 7.55-7.50 (m, 3H), 7.45-7.32 (m, 4H), 5.40 (s, 2H)

### Example 58

### 5-(2-Chlorophenyl)-1,2-dihydro-2-[(3-fluorophenyl)methyl]-3H-1,2,4-triazole-3-thione

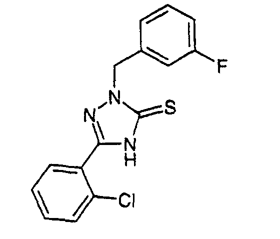

5-(2-Chlorophenyl)-3-triphenylmethylthio-1*H*-1,2,4-triazole (0.454g) (product of example 52, step a)), potassium carbonate (140mg) and 3-fluorophenylmethyl chloride (0.189g) were stirred together in dry DMF (1ml) for 3hrs. The mixture was poured onto water containing sodium chloride and extracted into dichloromethane. The extracts were washed with saturated sodium chloride solution and dried over magnesium sulfate. Trifluoroacetic acid (0.5ml) was added and the solution stood 15 mins. The solution was evaporated under reduced pressure. Purification was by chromatography eluting with dichloromethane. Trituration with ether/ isohexane gave a solid which was collected and dried. Yield 0.055g
m.p.: 125-126°C
MS: APCI(+ve): 320 (M+1)
¹H NMR: δ (DMSO) 14.05 (s, 1H), 7.66 (m, 2H), 7.59 (td, 1H), 7.47 (m, 2H), 7.17 (m, 3H), 5.41 (s, 2H)

### Example 59

### 5-(2-Chloraphenyl)-1,2-dihydro-2-[(2-iodophenyl)methyl]-3H-1,2,4-triazale-3-thione

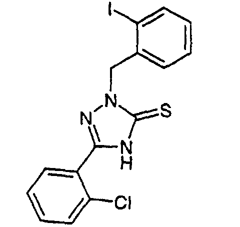

The title compound was prepared from 2-iodophenylmethyl bromide (0.252g) using the method of Example 58. Yield 0.14g.
m.p.: 189-190°C
MS: APCI(+ve): 428(M+1)
¹H NMR: δ (DMSO) 14.09 (s, 1H), 7.92 (dd, 1H), 7.69 (dd, 1H), 7.65 (dd, 1H),
7.59 (td, 1H), 7.50 (td, 1H), 7.39 (td, 1H), 7.09 (t,, 1H), 7.03 (d, 1H), 5.35 (s, 2H)

### Example 60

### 5-(2-Chlorophenyl)-1,2-dihydro-2-[(2,5-dimethylphenyl)methyl]-3H-1,2,4-triazole-3- thione

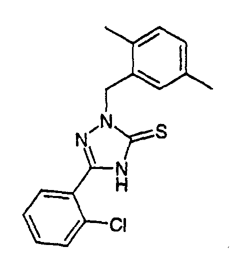

The title compound was prepared from 2,5-dimethylphenylmethyl bromide (0.155g) using the method of Example 58. Yield 0.152g.
m.p.: 166-167°C
MS: APCI(+ve): 330(M+1)
¹H NMR: δ (DMSO) 13.98 (s, 1H), 7.66 (m, 2H), 7.58 (td, 1H), 7.49 (td, 1H), 7.09 (d, 1H), 7.01 (d, 1H), 6.99 (s, 1H), 5.32 (s, 2H), 2.37 (s,3H), 2.22 (s, 3H)

### Example 61

### 5-(2-Chlorophenyl)-1,2-dihydro-2-[(2,5-difluorophenyl)methyl]-3H-1,2,4-triazole-3-thione

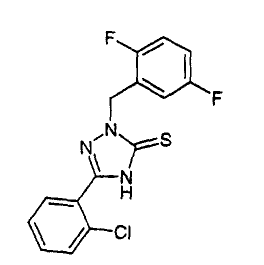

The title compound was prepared from 2,5-difluorophenylmethyl bromide (0.207g) using the method of Example 58. Yield 0.106g.
m.p.: 161-163°C
MS: APCI(+ve): 338(M+1)
¹H NMR: δ (DMSO) 14.07 (s, 1H), 7.70 (dd, 1H), 7.66 (dd, 1H), 7.59 (td, 1H), 7.50 (td, 1H), 7.32 (m, 1H), 7.24 (m, 1H), 7.17 (m, 1H), 5.43 (s, 2H)

### Example 62

### 2-[(3-Chlorophenyl)methyl]-5-(4-nitrophenyl)-1,2-dihydro-3H-1,2,4-triazole-3-thione

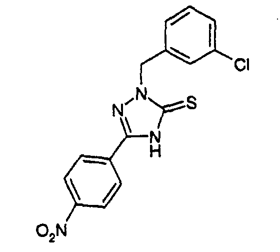

Prepared by the method of Example 5 using 4-nitrobenzoyl chloride (0.34g) and the product from Example 46 step (i) (0.40g). After acidification, the precipitate was collected by filtration. Purification was chromatography eluting with 20% ethyl acetate in isohexane followed by recrystallization from ethyl acetate / isohexane. Yield (0.095g).
m.p.: 256-258°C
MS: ESI(+ve) 347 (M+1, 100%)
¹H NMR: δ (DMSO) 14.49 (br, 1H), 8.38-8.35 (m, 2H), 8.18-8.14 (m, 2H), 7.47-7.34 (m, 4H), 5.43 (s, 2H)

### Example 63

### 2-[(3-Chlorophenyl)methyl]-5-(pyrimidin-5-yl)-1,2-dihydro-3H-1,2,4-triazole-3-thione

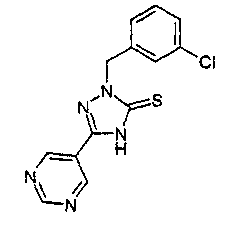

Prepared by the method of Example 5 using 5-pyrimidinecarbonyl chloride (0.13g) and the product from Example 46 step (i) (0.20g). After acidification, the precipitate was collected by filtration. Purification was chromatography eluting with methanol:ethyl acetate:dichloromethane (1:24:25) followed by chromatography eluting with methanol:ethyl acetate:isohexane (1:5:4) followed by trituration with diethyl ether. Yield (0.011g).
m.p.: 204-207°C
MS: APCI(-ve) 302 (M-1, 100%)
¹H NMR: δ (DMSO) 14.48 (br, 1H), 9.30 (s, 1H), 9.23 (s, 2H), 7.46-7.34 (m, 4H), 5.42 (s, 2H)

### Example 64

### 5-(2-Chlorophenyl)-1,2-dihydro-2-[(3-cyanophenyl)methyl]-3H-1,2,4-triazole-3-thione

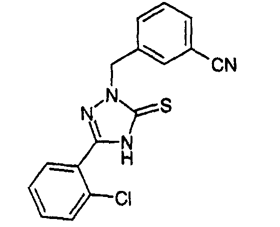

The title compound was prepared from 3-bromomethylbenonitrile (0.196g) using the method of Example 58. Crystallisation from ethyl acetate / isohexanes gave the title compound. Yield 0.049g.
m.p.: 161-163°C
MS: APCI(+ve): 327(M+1)
¹H NMR: δ (DMSO) 14.07 (s, 1H), 7.81 (bs, 2H), 7.69 (m, 2H), 7.59 (m, 3H),
7.50 (t, 1H), 5.46 (s, 2H)

### Example 65

### 5-(2-Chlorophenyl)-1,2-dihydro-2-[(4-nitrophenyl)methyl)-3H-1,2,4-triazole-3-thione

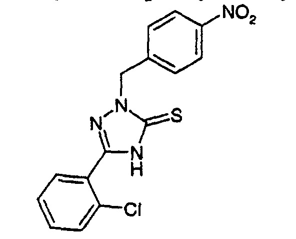

The title compound was prepared from 4-nitrophenylmethyl bromide (0.216g) using the method of Example 58. Yield 0.104g.
m.p.: 182-183°C
MS: APCI(+ve): 347(M+1)
¹H NMR: δ (DMSO) 14.11 (s, 1H), 8.24 (d, 2H), 7.67 (m, 2H), 7.61 (d, 2H), 7.51 (m, 2H), 5.55 (s, 2H)

### Example 66

### 5-(2-Chlorophenyl)-1,2-dihydro-2-(2-phenyl)ethyl-3H-1,2,4-triazole-3-thione

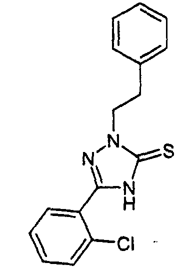

The title compound was prepared from (2-bromoethyl)benzene (0.185g) using the method of Example 58. Yield 0.093g.
m.p.: 131-133°C
MS: APCI(+ve): 316 (M+1)
¹H NMR: δ (DMSO) 13.85 (s, 1H), 7.63 (m, 3H), 7.50 (td, 1H), 7.22 (m, 5H), 4.37 (t, 2H), 3.12 (t, 2H).

### Example 67

### 5-(2-Chlorophenyl)-2-(2-(3-chlorophenyl)ethyl)-1,2-dihydro-3H-1,2,4-triazole-3-thione

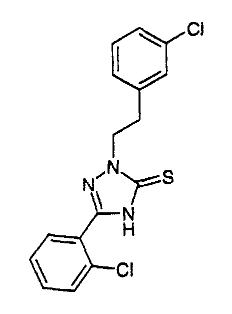

5-(2-Chlorophenyl)-3-triphenylmethylthio-1*H*-1,2,4-triazole (0.454g), triphenylphosphine (0.263g) and 3-chlorophenethylalcohol (0.155g) were stirred together in dry toluene (10ml). Diethylazodicarboxylate (0.175g) was added dropwise and the mixture stirred for 18hrs. Trifluoroacetic acid (1.0ml) was added and the mixture stirred 15mins. The solution was evaporated under reduced pressure. Purification was by chromatography eluting with dichloromethane to give an oil. Trituration with ether/ isohexane gave a solid, which was collected and dried. Yield 0.093g
m.p.: 132-134°C
MS: APCI(+ve): 351 (M+1)
¹H NMR: δ (DMSO) 13.85 (bs, 1H), 7.60 (m, 3H), 7.49 (td, 1H), 7.29 (m, 3H), 7.20 (dd, 1H), 4.39 (t, 2H), 3.15 (t, 2H)

### Example 68

### 5-(2-Chlorophenyl)-1,2-dihydro-2-(3-phenyl)propyl-3H-1,2,4-triazole-3-thione

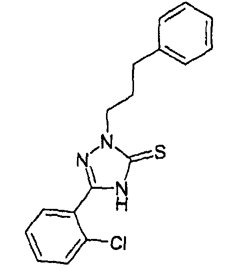

The title compound was prepared from 3-phenyl-1-propanol (0.136g) using the method of Example 58. Yield 0.080g.
m.p.: 80-82°C
MS: APCI(+ve): 330(M+1)
¹H NMR: δ (DMSO) 13.88 (bs, 1H), 7.67 (m, 2H), 7.59 (td, 1H), 7.50 (td, 1H), 7.25 (m, 5H), 4.16 (t, 2H), 2.66 (t, 2H), 2.10 (2H,m)

### Pharmacological Data

### Ligand Binding Assay

[¹²⁵I]IL-8 (human, recombinant) was purchased from Amersham, U.K. with a specific activity of 2,000Ci/mmol. All other chemicals were of analytical grade. High levels of hrCXCR2 were expressed in HEK 293 cells (human embryo kidney 293 cells ECACC No. 85120602) (Lee *et al.* (1992) *J. Biol. Chem.* **267** pp16283-16291). hrCXCR2 cDNA was amplified and cloned from human neutrophil mRNA. The DNA was cloned into PCRScript (Stratagene) and clones were identified using DNA. The coding sequence was sub-cloned into the eukaryotic expression vector RcCMV (Invitrogen). Plasmid DNA was prepared using Quiagen Megaprep 2500 and transfected into HEK 293 cells using Lipofectamine reagent (Gibco BRL). Cells of the highest expressing clone were harvested in phosphate-buffered saline containing 0.2%(w/v) ethylenediaminetetraacetic acid (EDTA) and centrifuged (200g, 5min.). The cell pellet was resuspended in ice cold homogenisation buffer [10mM HEPES (pH 7.4), 1mM dithiothreitol, 1mM EDTA and a panel of protease inhibitors (1mM phenyl methyl sulphonyl fluoride, 2µg/ml soybean trypsin inhibitor, 3mM benzamidine, 0.5µg/ml leupeptin and 100µg/ml bacitracin)] and the cells left to swell for 10 minutes. The cell preparation was disrupted using a hand held glass mortar/PTFE pestle homogeniser and cell membranes harvested by centrifugation (45 minutes, 100,000g, 4°C). The membrane preparation was stored at -70°C in homogenisation buffer supplemented with Tyrode's salt solution (137mM NaCl, 2.7mM KCl, 0.4mM NaH₂PO₄), 0.1%(w/v) gelatin and 10%(v/v) glycerol.

All assays were performed in a 96-well MultiScreen 0.45µm filtration plates (Millipore, U.K.). Each assay contained ∼33pM [¹²⁵I]IL-8 and membranes (equivalent to ∼80,000 cells) in assay buffer [Tyrode's salt solution supplemented with 10mM HEPES (pH 7.4), 1.8mM CaCl₂, 1mM MgCl₂, 0.5mg/ml bacitracin and 0.1%(w/v) gelatin]. In addition, a compound of formula (I) according to the Examples was pre-dissolved in DMSO and added to reach a final concentration of 1%(v/v) DMSO. The assay was initiated with the addition of membranes and after 1.5 hours at room temperature the membranes were harvested by filtration using a Millipore MultiScreen vacuum manifold and washed twice with assay buffer (without bacitracin). The backing plate was removed from the MultiScreen plate assembly, the filters dried at room temperature, punched out and then counted on a Cobra γ-counter.

The compounds of formula (I) according to the Examples were found to have IC₅₀ values of less than (<) 10µM.

### Intracellular Calcium Mobilisation Assay

Human neutrophils were prepared from EDTA-treated peripheral blood, as previously described (Baly *et al.* (1997) Methods in Enzymology 287 pp70-72), in storage buffer [Tyrode's salt solution (137mM NaCl, 2.7mM KCl, 0.4mM NaH₂PO₄) supplemented with 5.7mM glucose and 10mM HEPES (pH 7.4)].

The chemokine GROα (human, recombinant) was purchased from R&D Systems (Abingdon, U.K.). All other chemicals were of analytical grade. Changes in intracellular free calcium were measured fluorometrically by loading neutrophils with the calcium sensitive fluorescent dye, fluo-3, as described previously (Merritt *et al.* (1990) Biochem. J. 269, pp513-519). Cells were loaded for 1 hour at 37°C in loading buffer (storage buffer with 0.1%(w/v) gelatin) containing 5µM fluo-3 AM ester, washed with loading buffer and then resuspended in Tyrode's salt solution supplemented with 5.7mM glucose, 0.1%(w/v) bovine serum albumin (BSA), 1.8mM CaCl₂ and 1mM MgCl₂. The cells were pipetted into black walled, clear bottom, 96 well micro plates (Costar, Boston, U.S.A.) and centrifuged (200g, 5 minutes, room temperature).

A compound of formula (I) according to the Examples was pre-dissolved in DMSO and added to a final concentration of 0.1%(v/v) DMSO. Assays were initiated by the addition of an A₅₀ concentration of GROα and the transient increase in fluo-3 fluorescence (λ_{Ex} =490nm and λ_{Em} = 520nm) monitored using a FLIPR (Fluorometric Imaging Plate Reader, Molecular Devices, Sunnyvale, U.S.A.).

The compounds of formula (I) according to the Examples were tested and found to be antagonists of the CXCR2 receptor in human neutrophils.

## Claims

1. A compound of general formula 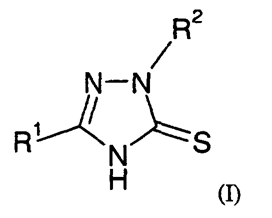 wherein R¹ represents a phenyl or naphthyl group, or a 5- or 6-membered heterocyclic aromatic group containing at least one heteroatom selected from nitrogen, oxygen and sulphur, each group being optionally substituted by one or more substituents independently selected from halogen atoms, nitro, cyano, hydroxyl, C₁-C₆ alkyl, C₁-C₆ alkoxy, C₁-C₆ alkoxycarbonyl, C₁-C₆ alkylthio, phenyl, phenoxy, phenylcarbonyl and trifluoromethyl groups; and
R² represents a C₁₋C₆ alkylphenyl group optionally substituted by one or more substituents independently selected from halogen atoms, hydroxyl, nitro, cyano, C₁-C₆ alkyl, C₁-C₆ alkoxy, phenoxy and trifluoromethyl groups,
with the provisos that:
• when R² represents an unsubstituted C₁ alkylphenyl group, then R¹ is not a phenyl, 4-chlorophenyl or 3-pyridinyl group,
• when R² represents an unsubstituted C₂ alkylphenyl group, then R¹ is not a phenyl group, and
• when R² represents a substituted C₁ alkylphenyl group, then it is not substituted by a hydroxyl group in the 2-position of the phenyl ring;
or a pharmaceutically acceptable salt or solvate thereof.

2. A compound according to claim 1, wherein for R¹, each group is optionally substituted by one or two substituents independently selected from C₁-C₄ alkyl, C₁-C₄ alkoxy, C₁-C₄ alkoxycarbonyl and C₁-C₄ alkylthio.

3. A compound according to claim 1 or claim 2, wherein R² represents a C₁₋C₄ alkylphenyl group optionally substituted by one or two substituents independently selected from halogen atoms, hydroxyl, nitro, cyano, C₁-C₄ alkyl, C₁-C₄ alkoxy, phenoxy and trifluoromethyl groups.

4. A compound according to any one of the preceding claims being selected from:
5-(2,5-Dichlorophenyl)-1,2-dihydro-2-(phenylmethyl)-3*H*-1,2,4-triazole-3-thione,
2-[1,2-Dihydro-1-(phenylmethyl)-5-thioxo-3*H*-1,2,4-triazol-3-yl]benzoic acid, methyl ester,
4-[1,2-Dihydro-1-(phenylmethyl)-5-thioxo-3*H*-1,2,4-triazol-3-yl]benzoic acid, methyl ester,
5-(5-Chloro-2-thienyl)-1,2-dihydro-2-(phenylmethyl)-3*H*-1,2,4-triazole-3-thione,
5-(3-Chlorophenyl)-1,2-dihydro-2-(phenylmethyl)-3*H*-1,2,4-triazole-3-thione,
1,2-Dihydro-5-(2-nitrophenyl)-2-(phenylmethyl)-3*H*-1,2,4-triazole-3-thione,
5-(4-Cyanophenyl)-1,2-dihydro-2-(phenylmethyl)-3*H*-1,2,4-triazole-3-thione,
5-(2,3-Dichlorophenyl)-1,2-dihydro-2-(phenylmethyl)-3*H*-1,2,4-triazole-3-thione,
5-(2-Bromophenyl)-1,2-dihydro-2-(phenylmethyl)-3*H*-1,2,4-triazole-3-thione,
1,2-Dihydro-2-(phenylmethyl)-5-[3-(trifluoromethyl)phenyl]-3*H*-1,2,4-triazole-3-thione,
5-(2-Furanyl)-1,2-dihydro-2-(phenylmethyl)-3*H*-1,2,4-triazole-3-thione,
5-(2-Bromo-5-methoxyphenyl)-1,2-dihydro-2-(phenylmethyl)-3*H*-1,2,4-triazole-3-thione,
5-(2,5-Dichloro-3-thienyl)-1,2-dihydro-2-(phenylmethyl)-3*H*-1,2,4-triazole-3-thione,
1,2-Dihydro-2-(phenylmethyl)-5-(4-pyridinyl)-3*H*-1,2,4-triazole-3-thione,
1,2-Dihydro-2-(phenylmethyl)-5-(4-phenylphenyl)-3*H*-1,2,4-triazole-3-thione,
5-(2-Chloro-3-pyridinyl)-1,2-dihydro-2-(phenylmethyl)-3*H*-1,2,4-triazole-3-thione,
5-(2-Fluorophenyl)-1,2-dihydro-2-(phenylmethyl)-3*H*-1,2,4-triazole-3-thione,
1,2-Dihydro-5-(2-methoxyphenyl)-2-(phenylmethyl)-3*H*-1,2,4-triazole-3-thione,
1,2-Dihydro-5-(2-methylphenyl)-2-(phenylmethyl)-3*H*-1,2,4-triazole-3-thione,
5-(3-Chloro-2-thienyl)-1,2-dihydro-2-(phenylmethyl)-3*H*-1,2,4-triazole-3-thione,
1,2-Dihydro-2-(phenylmethyl)-5-[4-(trifluoromethyl)phenyl]-3*H*-1,2,4-triazole-3-thione,
5-(2-Chloro-4-fluorophenyl)-1,2-dihydro-2-(phenylmethyl)-3*H*-1,2,4-triazole-3-thione,
1,2-Dihydro-5-(4-methoxyphenyl)-2-(phenylmethyl)-3*H*-1,2,4-triazole-3-thione,
1,2-Dihydro-5-(4-methylphenyl)-2-(phenylmethyl)-3*H*-1,2,4-triazole-3-thione,
5-(3,4-Dichlorophenyl)-1,2-dihydro-2-(phenylmethyl)-3*H*-1,2,4-triazole-3-thione,
5-(4-Bromophenyl)-1,2-dihydro-2-(phenylmethyl)-3*H*-1,2,4-triazole-3-thione,
5-(2,4-Dichlorophenyl)-1,2-dihydro-2-(phenylmethyl)-3*H*-1,2,4-triazole-3-thione,
5-(2-Chlorophenyl)-1,2-dihydro-2-(phenylmethyl)-3*H*-1,2,4-triazole-3-thione,
5-(3,5-Dichlorophenyl)-1,2-dihydro-2-(phenylmethyl)-3*H*-1,2,4-triazole-3-thione,
1,2-Dihydro-5-(3-phenoxyphenyl)-2-(phenylmethyl)-3*H*-1,2,4-triazole-3-thione,
1,2-Dihydro-2-(phenylmethyl)-5-(2-thienyl)-3*H*-1,2,4-triazole-3-thione,
2-[(3-Chlorophenyl)methyl]-5-(2,4-dichlorophenyl)-1,2-dihydro-3*H*-1,2,4-triazole-3-thione,
5-(2,4-Dichlorophenyl)-1,2-dihydro-2-[(3-methoxyphenyl)methyl]-3*H*-1,2,4-triazole-3-thione,
5-(2,4-Dichlorophenyl)-1,2-dihydro-2-[(3-phenoxyphenyl)methyl]-3*H*-1,2,4-triazole-3-thione,
1,2-Dihydro-2-[(3-hydroxyphenyl)methyl]-5-phenyl-3*H*-1,2,4-triazole-3-thione,
5-(2-Chloro-6-fluorophenyl)-1,2-dihydro-2-(phenylmethyl)-3*H*-1,2,4-triazole-3-thione,
1,2-Dihydro-5-(1-naphthalenyl)-2-(phenylmethyl)-3*H*-1,2,4-triazole-3-thione,
1,2-Dihydro-5-[2-(methylthio)phenyl]-2-(phenylmethyl)-3*H*-1,2,4-triazole-3-thione,
1,2-Dihydro-5-[2-(phenoxy)phenyl]-2-(phenylmethyl)-3*H*-1,2,4-triazole-3-thione,
1,2-Dihydro-5-(3-(phenylcarbonyl)phenyl)-2-(phenylmethyl)-3*H*-1,2,4-triazole-3-thione,
1,2-Dihydro-5-(1-bromo-2-naphthalenyl)-2-(phenylmethyl)-3*H*-1,2,4-triazole-3-thione,
5-(2-Chlorophenyl)-2-[(3-chlorophenyl)methyl]-1,2-dihydro-3*H*-1,2,4-triazole-3-thione,
5-(2,4-Dichlorophenyl)-1,2-dihydro-2-[(3-methylphenyl)methyl]-3*H*-1,2,4-triazole-3-thione,
5-(2,4-Dichlorophenyl)-1,2-dihydro-2-(2-phenylethyl)-3*H*-1,2,4-triazole-3-thione,
2-[(4-Chlorophenyl)methyl]-5-(2,4-dichlorophenyl)-1,2-dihydro-3*H*-1,2,4-triazole-3-thione,
5-(4-Bromophenyl)-2-[(3-chlorophenyl)methyl]-1,2-dihydro-3*H*-1,2,4-triazole-3-thione,
2-[(3-Chlorophenyl)methyl]-5-(4-methylphenyl)-1,2-dihydro-3*H*-1,2,4-triazole-3-thione,
2-[(3-Chlorophenyl)methyl]-5-(4-methoxyphenyl)-1,2-dihydro-3*H*-1,2,4-triazole-3-thione,
2-[(3-Chlorophenyl)methyl]-5-[4-(1-methylethyl)phenyl]-1,2-dihydro-3*H*-1,2,4-triazole-3-thione,
5-(2-Chlorophenyl)-1,2-dihydro-2-(4-methylphenyl)methyl-3*H*-1,2,4-triazole-3-thione,
5-(2-Chlorophenyl)-1,2-dihydro-2-[(4-(1,1-dimethylethyl)phenyl)methyl)-3*H*-1,2,4-triazole-3-thione,
5-(2-Chlorophenyl)-1,2-dihydro-2-[(3-phenyloxy)phenylmethyl]-3*H*-1,2,4-triazole-3-thione,
5-(2-Chlorophenyl)-1,2-dihydro-2-[[4-(trifluoromethyl)phenyl]methyl]-3*H*-1,2,4-triazole-3-thione,
5-(2-Chlorophenyl)-1,2-dihydro-2-[(4-(methoxyphenyl)methyl]-3*H*-1,2,4-triazole-3-thione,
2-[(3-Chlorophenyl)methyl]-5-(4-phenoxyphenyl)-1,2-dihydro-3*H*-1,2,4-triazole-3-thione,
2-[(3-Chlorophenyl)methyl]-5-(3-pyridinyl)-1,2-dihydro-3*H*-1,2,4-triazole-3-thione,
2-[(3-Chlorophenyl)methyl]-5-phenyl-1,2-dihydro-3*H*-1,2,4-triazole-3-thione,
5-(2-Chlorophenyl)-1,2-dihydro-2-[(3-fluorophenyl)methyl]-3*H*-1,2,4-triazole-3-thione,
5-(2-Chlorophenyl)-1,2-dihydro-2-[(2-iodophenyl)methyl]-3*H*-1,2,4-triazole-3-thione,
5-(2-Chlorophenyl)-1,2-dihydro-2-[(2,5-dimethylphenyl)methyl]-3*H*-1,2,4-triazole-3-thione,
5-(2-Chlorophenyl)-1,2-dihydro-2-[(2,5-difluorophenyl)methyl]-3*H*-1,2,4-triazole-3-thione,
2-[(3-Chlorophenyl)methyl]-5-(4-nitrophenyl)-1,2-dihydro-3*H*-1,2,4-triazole-3-thione,
2-[(3-Chlorophenyl)methyl]-5-(pyrimidin-5-yl)-1,2-dihydro-3*H*-1,2,4-triazole-3-thione,
5-(2-Chlorophenyl)-1,2-dihydro-2-[(3-cyanophenyl)methyl]-3*H*-1,2,4-triazole-3-thione,
5-(2-Chlorophenyl)-1,2-dihydro-2-[(4-nitrophenyl)methyl]-3*H*-1,2,4-triazole-3-thione,
5-(2-Chlorophenyl)-1,2-dihydro-2-(2-phenyl)ethyl-3*H*-1,2,4-triazole-3-thione,
5-(2-Chlorophenyl)-2-(2-(3-chlorophenyl)ethyl)-1,2-dihydro-3*H*-1,2,4-triazole-3-thione,
5-(2-Chlorophenyl)-1,2-dihydro-2-(3-phenyl)propyl-3*H*-1,2,4-triazole-3-thione,
and their pharmaceutically acceptable salts and solvates.

5. A process for preparing a compound of formula (I) as defined in claim 1 which comprises:
(a) reacting a compound of general formula (II), R¹-C(O)L, wherein L represents a leaving group and R¹ is as defined in formula (I), with a compound of general formula 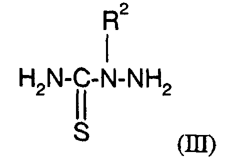 wherein R² is as defined in formula (I), followed by cyclisation; or
(b) reacting a compound of general formula 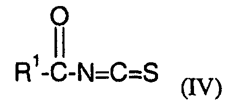 wherein R¹ is as defined in formula (I), with a compound of general formula
R²-NHNH₂ (V)
wherein R² is as defined in formula (I), followed by cyclisation; or
(c) reacting a compound of general formula 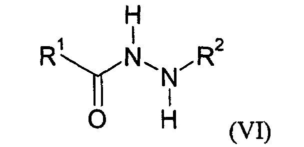 wherein R¹ and R² are as defined in formula (I), with ammonium thiocyanate, followed by cyclisation; or
(d) reacting a compound of general formula 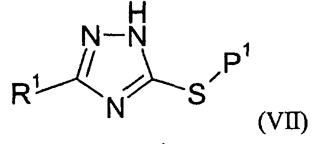
wherein P¹ represents a protecting group and R¹ is as defined in formula (I), with a compound of general formula (VIII), R² - L¹, wherein L¹ represents a leaving group and R² is as defined in formula (I), followed by removal of the protecting group P¹;
and optionally after (a), (b), (c) or (d) forming a pharmaceutically acceptable salt or solvate of the compound of formula (I).

6. A compound of formula (VII) as defined in claim 5.

7. A pharmaceutical composition comprising a compound of formula (I), or a pharmaceutically acceptable salt or solvate thereof, as claimed in any one of claims 1 to 4 in association with a pharmaceutically acceptable adjuvant, diluent or carrier.

8. A process for the preparation of a pharmaceutical composition as claimed in claim 7 which comprises mixing a compound of formula (I), or a pharmaceutically acceptable salt or solvate thereof, as claimed in any one of claims 1 to 4 with a pharmaceutically acceptable adjuvant, diluent or carrier.

9. A compound of formula (I), or a pharmaceutically-acceptable salt or solvate thereof, as claimed in any one of claims 1 to 4 for use in therapy.

10. Compound for use according to claim 9 wherein the disease is psoriasis.

## Patentansprüche

1. Verbindung der allgemeinen Formel 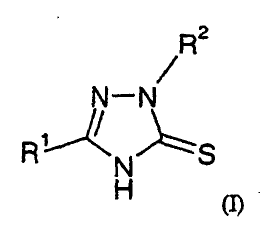 worin R¹ für eine Phenyl- oder Naphthylgruppe oder eine 5- oder 6-gliedrige heterocyclische aromatische Gruppe mit mindestens einem unter Stickstoff, Sauerstoff und Schwefel ausgewählten Heteroatom steht, wobei jede Gruppe gegebenenfalls durch einen oder mehrere, unabhängig voneinander unter Halogenatomen und Nitro-, Cyano-, Hydroxyl-, C₁-C₆-Alkyl-, C₁-C₆-Alkoxy-, C₁-C₆-Alkoxycarbonyl-, C₁-C₆-Alkylthio-, Phenyl-, Phenoxy-, Phenylcarbonyl- und Trifluormethylgruppen ausgewählte Substituenten substituiert ist; und
R² für eine gegebenenfalls durch einen oder mehrere, unabhängig voneinander unter Halogenatomen und Hydroxyl-, Nitro-, Cyano-, C₁₋C₆-Alkyl-, C₁-C₆-Alkoxy-, Phenoxy- und Trifluormethylgruppen ausgewählte Substituenten substituierte C₁-C₆-Alkylphenylgruppe steht,
mit den Maßgaben, daß:
• R¹ nicht für eine Phenyl-, 4-Chlorphenyl- oder 3-Pyridinylgruppe steht, wenn R² für eine unsubstituierte C₁-Alkylphenylgruppe steht,
• R¹ nicht für eine Phenylgruppe steht, wenn R² für eine unsubstituierte C₂-Alkylphenylgruppe steht, und
• R² für den Fall, daß dieser Substituent für eine substituierte C₁-Alkylphenylgruppe steht, nicht in der 2-Stellung des Phenylrings durch eine Hydroxylgruppe substituiert ist;
oder ein pharmazeutisch unbedenkliches Salz oder Solvat davon.

2. Verbindung nach Anspruch 1, worin für R¹ jede Gruppe gegebenenfalls durch einen oder zwei, unabhängig voneinander unter C₁-C₄-Alkyl-, C₁-C₄-Alkoxy-, C₁-C₄-Alkoxycarbonyl- und C₁-C₄-Alkylthio ausgewählte Substituenten substituiert ist.

3. Verbindung nach Anspruch 1 oder 2, worin R² für eine gegebenenfalls durch einen oder zwei, unabhängig voneinander unter Halogenatomen und Hydroxyl-, Nitro-, Cyano-, C₁₋C₄-Alkyl-, C₁-C₄-Alkoxy-, Phenoxy- und Trifluormethylgruppen ausgewählte Substituenten substituierte C₁-C₄-Alkylphenylgruppe steht.

4. Verbindung nach einem der vorhergehenden Ansprüche, ausgewählt unter:
5-(2,5-Dichlorphenyl)-1,2-dihydro-2-(phenylmethyl)-3*H*-1,2,4-triazol-3-thion,
2-[1,2-Dihydro-1-(phenylmethyl)-5-thioxo-3*H*-1,2,4-triazol-3-yl]benzoesäuremethylester,
4-[1,2-Dihydro-1-(phenylmethyl)-5-thioxo-3*H*-1,2,4-triazol-3-yl]benzoesäuremethylester,
5-(5-Chlor-2-thienyl)-1,2-dihydro-2-(phenylmethyl)-3*H*-1,2,4-triazol-3-thion,
5-(3-Chlorphenyl)-1,2-dihydro-2-(phenylmethyl)-3*H*-1,2,4-triazol-3-thion,
1,2-Dihydro-5-(2-nitrophenyl)-2-(phenylmethyl)-3*H*-1,2,4-triazol-3-thion,
5-(4-Cyanophenyl)-1,2-dihydro-2-(phenylmethyl)-3*H*-1,2,4-triazol-3-thion,
5-(2,3-Dichlorphenyl)-1,2-dihydro-2-(phenylmethyl) -3*H*-1,2,4-triazol-3-thion,
5-(2-Bromphenyl)-1,2-dihydro-2-(phenylmethyl)-3*H*-1,2, 4-triazol-3-thion,
1,2-Dihydro-2-(phenylmethyl)-5-[3-(trifluormethyl)phenyl]-3*H*-1,2,4-triazol-3-thion,
5-(2-Furanyl) -1, 2-dihydro-2- (phenylmethyl) -3*H*-1,2,4-triazol-3-thion,
5-(2-Brom-5-methoxyphenyl)-1,2-dihydro-2-(phenylmethyl)-3*H*-1,2,4-triazol-3-thion,
5-(2,5-Dichlor-3-thienyl)-1,2-dihydro-2-(phenylmethyl) -3*H*-1,2,4-triazol-3-thion,
1,2-Dihydro-2-(phenylmethyl)-5-(4-pyridinyl)-3*H*-1,2,4-triazol-3-thion,
1,2-Dihydro-2-(phenylmethyl)-5-(4-phenylphenyl)-3*H*-1,2,4-triazol-3-thion,
5-(2-Chlor-3-pyridinyl)-1,2-dihydro-2-(phenylmethyl) -3*H*-1,2,4-triazol-3-thion,
5-(2-Fluorphenyl)-1,2-dihydro-2-(phenylmethyl)-3*H*-1,2,4-triazol-3-thion,
1,2-Dihydro-5-(2-methoxyphenyl)-2-(phenylmethyl)-3*H*-1,2,4-triazol-3-thion,
1,2-Dihydro-5-(2-methylphenyl)-2-(phenylmethyl)-3*H*-1,2,4-triazol-3-thion,
5-(3-Chlor-2-thienyl)-1,2-dihydro-2-(phenylmethyl)-3*H*-1,2,4-triazol-3-thion,
1,2-Dihydro-2-(phenylmethyl)-5-[4-(trifluormethyl)phenyl]-3*H*-1,2,4-triazol-3-thion,
5-(2-Chlor-4-fluorphenyl)-1,2-dihydro-2-(phenylmethyl)-3*H*-1,2,4-triazol-3-thion,
1,2-Dihydro-5-(4-methoxyphenyl)-2-(phenylmethyl)-3*H*-1,2,4-triazol-3-thion,
1,2-Dihydro-5-(4-methylphenyl)-2-(phenylmethyl)-3*H*-1,2,4-triazol-3-thion,
5-(3,4-Dichlorphenyl)-1,2-dihydro-2-(phenylmethyl)-3*H*-1,2,4-triazol-3-thion,
5-(4-Bromphenyl)-1,2-dihydro-2-(phenylmethyl)-3*H*-1,2,4-triazol-3-thion,
5-(2,4-Dichlorphenyl)-1,2-dihydro-2-(phenylmethyl) -3*H*-1,2,4-triazol-3-thion,
5-(2-Chlorphenyl)-1,2-dihydro-2-(phenylmethyl)-3*H*-1,2,4-triazol-3-thion,
5-(3,5-Dichlorphenyl)-1,2-dihydro-2-(phenylmethyl)-3*H*-1,2,4-triazol-3-thion,
1,2-Dihydro-5-(3-phenoxyphenyl)-2-(phenylmethyl)-3*H*-1,2,4-triazol-3-thion,
1,2-Dihydro-2-(phenylmethyl)-5-(2-thienyl)-3*H*-1,2,4-triazol-3-thion,
2-[(3-Chlorphenyl)methyl]-5-(2,4-dichlorphenyl)-1,2-dihydro-3*H*-1,2,4-triazol-3-thion,
5-(2,4-Dichlorphenyl)-1,2-dihydro-2-[(3-methoxyphenyl)methyl]-3*H*-1,2,4-triazol-3-thion,
5-(2,4-Dichlorphenyl)-1,2-dihydro-2-[(3-phenoxyphenyl)methyl]-3*H*-1,2,4-triazol-3-thion,
1,2-Dihydro-2-[(3-hydroxyphenyl)methyl]-5-phenyl-3*H*-1,2,4-triazol-3-thion,
5-(2-Chlor-6-fluorphenyl)-1,2-dihydro-2-(phenylmethyl)-3*H*-1,2,4-triazol-3-thion,
1,2-Dihydro-5-(1-naphthalinyl)-2-(phenylmethyl)-3*H*-1,2,4-triazol-3-thion,
1,2-Dihydro-5-[2-(methylthio)phenyl]-2-(phenylmethyl) -3*H*-1,2,4-triazol-3-thion,
1,2-Dihydro-5-[2-(phenoxy)phenyl]-2-(phenylmethyl) -3*H*-1,2,4-triazol-3-thion,
1,2-Dihydro-5-((3-phenylcarbonyl)phenyl)-2-(phenylmethyl)-3*H*-1,2,4-triazol-3-thion,
1,2-Dihydro-5-(1-brom-2-naphthalinyl)-2-(phenylmethyl)-3*H*-1,2,4-triazol-3-thion,
5-(2-Chlorphenyl)-2-[(3-chlorphenyl)methyl]-1,2-dihydro-3*H*-1,2,4-triazol-3-thion,
5-(2,4-Dichlorphenyl)-1,2-dihydro-2-[(3-methylphenyl)methyl]-3*H*-1,2,4-triazol-3-thion,
5-(2,4-Dichlorphenyl)-1,2-dihydro-2-(2-phenylethyl)-3*H*-1,2,4-triazol-3-thion,
2-[(4-Chlorphenyl)methyl]-5-(2,4-dichlorphenyl)-1,2-dihydro-3*H*-1,2,4-triazol-3-thion,
5-(4-Bromphenyl)-2-[(3-chlorphenyl)methyl]-1,2-dihydro-3*H*-1,2,4-triazol-3-thion,
2-[(3-Chlorphenyl)methyl]-5-(4-methylphenyl)-1,2-dihydro-3*H*-1,2,4-triazol-3-thion,
2-[(3-Chlorphenyl)methyl]-5-(4-methoxyphenyl)-1,2-dihydro-3*H*-1,2,4-triazol-3-thion,
2-[(3-Chlorphenyl)methyl]-5-[4-(1-methylethyl)-phenyl]-1,2-dihydro-3*H*-1,2,4-triazol-3-thion,
5-(2-Chlorphenyl)-1,2-dihydro-2-(4-methylphenyl)-methyl-3*H*-1,2,4-triazol-3-thion,
5-(2-Chlorphenyl)-1,2-dihydro-2-[(4-(1,1-dimethylethyl)phenyl)methyl]-3*H*-1,2,4-triazol-3-thion,
5-(2-Chlorphenyl)-1,2-dihydro-2-[(3-phenyloxy)-phenylmethyl]-3*H*-1,2,4-triazol-3-thion,
5-(2-Chlorphenyl)-1,2-dihydro-2-[[4-(trifluormethyl)phenyl]methyl]-3*H*-1,2,4-triazol-3-thion,
5-(2-Chlorphenyl)-1,2-dihydro-2-[(4-(methoxyphenyl)methyl]-3*H*-1,2,4-triazol-3-thion,
2-[(3-Chlorphenyl)methyl]-5-(4-phenoxyphenyl)-1,2-dihydro-3*H*-1,2,4-triazol-3-thion,
2-[(3-Chlorphenyl)methyl]-5-(3-pyridinyl)-1,2-dihydro-3*H*-1,2,4-triazol-3-thion,
2-[(3-Chlorphenyl)methyl]-5-phenyl-1,2-dihydro-3*H*-1,2,4-triazol-3-thion,
5-(2-Chlorphenyl)-1,2-dihydro-2-[(3-fluorphenyl)-methyl]-3*H*-1,2,4-triazol-3-thion,
5-(2-Chlorphenyl)-1,2-dihydro-2-[(2-iodphenyl)-methyl]-3*H*-1,2,4-triazol-3-thion,
5-(2-Chlorphenyl)-1,2-dihydro-2-[(2,5-dimethylphenyl)methyl]-3*H*-1,2,4-triazol-3-thion,
5-(2-Chlorphenyl)-1,2-dihydro-2-[(2,5-difluorphenyl)methyl]-3*H*-1,2,4-triazol-3-thion,
2-[(3-Chlorphenyl)methyl]-5-(4-nitrophenyl)-1,2-dihydro-3*H*-1,2,4-triazol-3-thion,
2-[(3-Chlorphenyl)methyl]-5-(pyrimidin-5-yl)-1,2-dihydro-3*H*-1,2,4-triazol-3-thion,
5-(2-Chlorphenyl)-1,2-dihydro-2-[(3-cyanophenyl)-methyl]-3*H*-1,2,4-triazol-3-thion,
5-(2-Chlorphenyl)-1,2-dihydro-2-[(4-nitrophenyl)-methyl]-3*H*-1,2,4-triazol-3-thion,
5-(2-Chlorphenyl)-1,2-dihydro-2-(2-phenyl)ethyl-3*H*-1,2,4-triazol-3-thion,
5-(2-Chlorphenyl)-2-(2-(3-chlorphenyl)ethyl)-1,2-dihydro-3*H*-1,2,4-triazol-3-thion,
5-(2-Chlorphenyl)-1,2-dihydro-2-(3-phenyl)propyl-3*H*-1,2,4-triazol-3-thion,
und pharmazeutisch unbedenklichen Salzen und Solvaten davon.

5. Verfahren zur Herstellung einer Verbindung der Formel (I) nach Anspruch 1, bei dem man:
(a) eine Verbindung der allgemeinen Formel (II), R¹-C(O)L, worin L für eine Abgangsgruppe steht und R¹ die unter Formel (I) angegebene Bedeutung besitzt, mit einer Verbindung der allgemeinen Formel 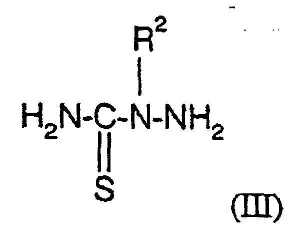 worin R² die unter Formel (I) angegebene Bedeutung besitzt, umsetzt und danach cyclisiert; oder
(b) eine Verbindung der allgemeinen Formel 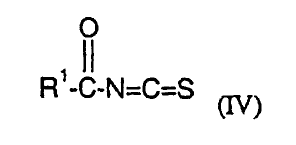 worin R¹ die unter Formel (I) angegebene Bedeutung besitzt, mit einer Verbindung der allgemeinen Formel
R²-NHNH₂ (V)
worin R² die unter Formel (I) angegebene Bedeutung besitzt, umsetzt und danach cyclisiert; oder
(c) eine Verbindung der allgemeinen Formel 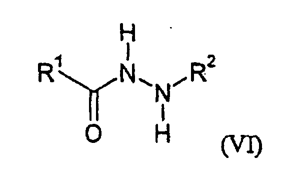 worin R¹ und R² die unter Formel (I) angegebene Bedeutung besitzen, mit Ammoniumthiocyanat umsetzt und danach cyclisiert; oder
(d) eine Verbindung der allgemeinen Formel 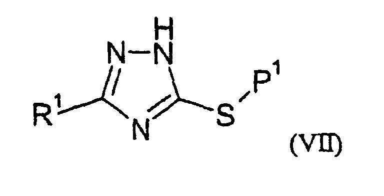
worin P¹ für eine Schutzgruppe steht und R¹ die unter Formel (I) angegebene Bedeutung besitzt, mit einer Verbindung der allgemeinen Formel (VIII), R²-L¹, worin L¹ für eine Abgangsgruppe steht und R² die unter Formel (I) angegebene Bedeutung besitzt, umsetzt und danach die Schutzgruppe P¹ abspaltet;
und gegebenenfalls nach (a), (b), (c) oder (d) ein pharmazeutisch unbedenkliches Salz oder Solvat der Verbindung der Formel (I) herstellt.

6. Verbindung der Formel (VII) nach Anspruch 5.

7. Pharmazeutische Zusammensetzung, enthaltend eine Verbindung der Formel (I) oder ein pharmazeutisch unbedenkliches Salz oder Solvat davon nach einem der Ansprüche 1 bis 4 zusammen mit einem pharmazeutisch unbedenklichen Hilfsstoff, Verdünnungsmittel oder Träger.

8. Verfahren zur Herstellung einer pharmazeutischen Zusammensetzung nach Anspruch 7, bei dem man eine Verbindung der Formel (I) oder ein pharmazeutisch unbedenkliches Salz oder Solvat davon nach einem der Ansprüche 1 bis 4 mit einem pharmazeutisch unbedenklichen Hilfsstoff, Verdünnungsmittel oder Träger vermischt.

9. Verbindung der Formel (I) oder ein pharmazeutisch unbedenkliches Salz oder Solvat davon nach einem der Ansprüche 1 bis 4 zur Verwendung bei der Therapie.

10. Verbindung zur Verwendung nach Anspruch 9, wobei es sich bei der Krankheit um Psoriasis handelt.

## Revendications

1. Composé de formule générale 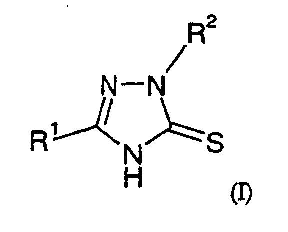 dans laquelle R¹ représente un groupement phényle ou naphtyle, ou un groupement aromatique hétérocyclique à 5 ou 6 chaînons contenant au moins un hétéroatome choisi parmi l'azote, l'oxygène et le soufre, chaque groupement étant éventuellement substitué par un ou plusieurs substituants choisis indépendamment parmi les atomes d'halogène et les groupements nitro, cyano, hydroxy, alkyle en C₁-C₆, alcoxy en C₁-C₆, alcoxycarbonyle en C₁-C₆, alkylthio en C₁-C₆, phényle, phénoxy, phénylcarbonyle et trifluorométhyle ; et
R² représente un groupement alkylphényle en C₁₋C₆ substitué éventuellement par un ou plusieurs substituants choisis indépendamment parmi les atomes d'halogène et les groupements hydroxy, nitro, cyano, alkyle en C₁-C₆, alcoxy en C₁-C₆, phénoxy et trifluorométhyle,
à condition que :
• lorsque R² représente un groupement alkylphényle en C₁ non substitué, alors R¹ n'est pas un groupement phényle, 4-chlorophényle ou 3-pyridinyle,
• lorsque R² représente un groupement alkylphényle en C₂ non substitué, alors R¹ n'est pas un groupement phényle, et
• lorsque R² représente un groupement alkylphényle en C₁ substitué, alors il n'est pas substitué par un groupement hydroxy en position 2 du noyau phényle ;
ou un sel pharmaceutiquement acceptable ou un solvate de celui-ci.

2. Composé selon la revendication 1, **caractérisé en** de ce que pour R¹, chaque groupement est éventuellement substitué par un ou deux substituants choisis indépendamment parmi alkyle en C₁-C₄, alcoxy en C₁-C₄, alcoxycarbonyle en C₁-C₄ et alkylthio en C₁-C₄.

3. Composé selon la revendication 1 ou la revendication 2, **caractérisé en ce que** R² représente un groupement alkylphényle en C₁-C₄, éventuellement substitué par un ou deux substituants choisis indépendamment parmi les atomes d'halogène et les groupements hydroxy, nitro, cyano, alkyle en C₁-C₄, alcoxy en C₁-C₄, phénoxy et trifluorométhyle.

4. Composé selon l'une quelconque des revendications précédentes, étant choisi parmi :
la 5-(2,5-dichlorophényl)-1,2-dihydro-2-(phénylméthyl)-3*H*-1,2,4-triazole-3-thione,
l'ester méthylique de l'acide 2-[1,2-dihydro-1-(phénylméthyl)-5-thioxo-3*H*-1,2,4-triazole-3-yl]benzoïque,
l'ester méthylique de l'acide 4-[1,2-dihydro-1-(phénylméthyl)-5-thioxo-3*H*-1,2,4-triazole-3-yl]benzoïque,
la 5-(5-chloro-2-thiényl)-1,2-dihydro-2-(phénylméthyl)-3*H*-1,2,4-triazole-3-thione,
la 5-(3-chlorophényl)-1,2-dihydro-2-(phénylméthyl)-3*H*-1,2,4-triazole-3-thione,
la 1,2-dihydro-5-(2-nitrophényl)-2-(phénylméthyl)-3*H*-1,2,4-triazole-3-thione,
la 5-(4-cyanophényl)-1,2-dihydro-2-(phénylméthyl)-3*H*-1,2,4-triazole-3-thione,
la 5-(2,3-dichlorophényl)-1,2-dihydro-2-(phénylméthyl)-3*H*-1,2,4-triazole-3-thione,
la 5-(2-bromophényl)-1,2-dihydro-2-(phénylméthyl)-3*H*-1,2,4-triazole-3-thione,
la 1,2-dihydro-2-(phénylméthyl)-5-[3-(trifluorométhyl)phényl]-3*H*-1,2,4-triazole-3-thione,
la 5-(2-furanyl)-1,2-dihydro-2-(phénylméthyl)-3*H*-1,2,4-triazole-3-thione,
la 5-(2-bromo-5-méthoxyphényl)-1,2-dihydro-2-(phénylméthyl)-3*H*-1,2,4-triazole-3-thione,
la 5-(2,5-dichloro-3-thiényl)-1,2-dihydro-2-(phénylméthyl)-3*H*-1,2,4-triazole-3-thione,
la 1,2-dihydro-2-(phénylméthyl)-5-(4-pyridinyl)-3*H*-1,2,4-triazole-3-thione,
la 1,2-dihydro-2-(phénylméthyl)-5-(4-phénylphényl)-3*H*-1,2,4-triazole-3-thione,
la 5-(2-chloro-3-pyridinyl)-1,2-dihydro-2-(phénylméthyl) -3*H*-1,2,4-triazole-3-thione,
la 5-(2-fluorophényl)-1,2-dihydro-2-(phénylméthyl) -3*H*-1,2,4-triazole-3-thione,
la 1,2-dihydro-5-(2-méthoxyphényl)-2-(phénylméthyl)-3*H*-1,2,4-triazole-3-thione,
la 1,2-dihydro-5-(2-méthylphényl)-2-(phénylméthyl)-3*H*-1,2,4-triazole-3-thione,
la 5-(3-chloro-2-thiényl)-1,2-dihydro-2-(phénylméthyl) -3*H*-1,2,4-triazole-3-thione,
la 1,2-dihydro-2-(phénylméthyl)-5-[4-(trifluorométhyl)phényl]-3*H*-1,2,4-triazole-3-thione,
la 5-(2-chloro-4-fluorophényl)-1,2-dihydro-2-(phénylméthyl)-3*H*-1,2,4-triazole-3-thione,
la 1,2-dihydro-5-(4-méthoxyphényl)-2-(phénylméthyl)-3*H*-1,2,4-triazole-3-thione,
la 1,2-dihydro-5-(4-méthylphényl)-2-(phénylméthyl)-3*H*-1,2,4-triazole-3-thione,
la 5-(3,4-dichlorophényl)-1,2-dihydro-2-(phénylméthyl)-3*H*-1,2,4-triazole-3-thione,
la 5-(4-bromophényl)-1,2-dihydro-2-(phénylméthyl)-3*H*-1,2,4-triazole-3-thione,
la 5-(2,4-dichlorophényl)-1,2-dihydro-2-(phénylméthyl)-3*H*-1,2,4-triazole-3-thione,
la 5-(2-chlorophényl)-1,2-dihydro-2-(phénylméthyl)-3*H*-1,2,4-triazole-3-thione,
la 5-(3,5-dichlorophényl)-1,2-dihydro-2-(phénylméthyl)-3*H*-1,2,4-triazole-3-thione,
la 1,2-dihydro-5-(3-phénoxyphényl)-2-(phénylméthyl)-3*H*-1,2,4-triazole-3-thione,
la 1,2-dihydro-2-(phénylméthyl)-5-(2-thiényl)-3*H*-1,2,4-triazole-3-thione,
la 2-[(3-chlorophényl)méthyl]-5-(2,4-dichlorophényl)-1,2-dihydro-3*H*-1,2,4-triazole-3-thione,
la 5-(2,4-dichlorophényl)-1,2-dihydro-2-[(3-méthoxyphényl)méthyl]-3*H*-1,2,4-triazole-3-thione,
la 5-(2,4-dichlorophényl)-1,2-dihydro-2-[(3-phénoxyphényl)méthyl]-3*H*-1,2,4-triazole-3-thione,
la 1,2-dihydro-2-[(3-hydroxyphényl)méthyl]-5-phényl-3*H*-1,2,4-triazole-3-thione,
la 5-(2-chloro-6-fluorophényl)-1,2-dihydro-2-(phénylméthyl)-3*H*-1,2,4-triazole-3-thione,
la 1,2-dihydro-5-(1-naphtalényl)-2-(phénylméthyl)-3*H*-1,2,4-triazole-3-thione,
la 1,2-dihydro-5-[2-(méthylthio)phényl]-2-(phénylméthyl)-3*H*-1,2,4-triazole-3-thione,
la 1,2-dihydro-5-[2-(phénoxy)phényl]-2-(phénylméthyl) -3*H*-1,2,4-triazole-3-thione,
la 1,2-dihydro-5-(3-(phénylcarbonyl)phényl)-2-(phénylméthyl)-3*H*-1,2,4-triazole-3-thione,
la 1,2-dihydro-5-(1-bromo-2-naphtalényl)-2-(phénylméthyl)-3*H*-1,2,4-triazole-3-thione,
la 5-(2-chlorophényl)-2-[(3-chlorophényl)méthyl]-1,2-dihydro-3*H*-1,2,4-triazole-3-thione,
la 5-(2,4-dichlorophényl)-1,2-dihydro-2-[(3-méthylphényl)méthyl]-3*H*-1,2,4-triazole-3-thione,
la 5-(2,4-dichlorophényl)-1,2-dihydro-2-(2-phényléthyl)-3*H*-1,2,4-triazole-3-thione,
la 2-[(4-chlorophényl)méthyl]-5-(2,4-dichlorophényl) -1,2-dihydro-3*H*-1,2,4-triazole-3-thione,
la 5-(4-bromophényl)-2-[(3-chlorophényl)méthyl]-1,2-dihydro-3*H*-1,2,4-triazole-3-thione,
la 2-[(3-chlorophényl)méthyl]-5-(4-méthylphényl)-1,2-dihydro-3*H*-1,2,4-triazole-3-thione,
la 2-[(3-chlorophényl)méthyl]-5-(4-méthoxyphényl)-1,2-dihydro-3*H*-1,2,4-triazole-3-thione,
la 2-[(3-chlorophényl)méthyl]-5-[4-(1-méthyléthyl)phényl]-1,2-dihydro-3*H*-1,2,4-triazole-3-thione,
la 5-(2-chlorophényl)-1,2-dihydro-2-(4-méthylphényl)méthyl-3*H*-1,2,4-triazole-3-thione,
la 5-(2-chlorophényl)-1,2-dihydro-2-[(4-(1,1-diméthyléthyl)phényl)méthyl]-3*H*-1,2,4-triazole-3-thione,
la 5-(2-chlorophényl)-1,2-dihydro-2-[(3-phényloxy)phénylméthyl]-3*H*-1,2,4-triazole-3-thione,
la 5-(2-chlorophényl)-1,2-dihydro-2-[[4-(trifluorométhyl)phényl]méthyl]-3*H*-1,2,4-triazole-3-thione,
la 5-(2-chlorophényl)-1,2-dihydro-2-[4-(méthoxyphényl)méthyl]-3*H*-1,2,4-triazole-3-thione,
la 2-[(3-chlorophényl)méthyl]-5-(4-phénoxyphényl)-1,2-dihydro-3*H*-1,2,4-triazole-3-thione,
la 2-[(3-chlorophényl)méthyl]-5-(3-pyridinyl)-1,2-dihydro-3*H*-1,2,4-triazole-3-thione,
la 2-[(3-chlorophényl)méthyl]-5-phényl-1,2-dihydro-3*H*-1,2,4-triazole-3-thione,
la 5-(2-chlorophényl)-1,2-dihydro-2-[(3-fluorophényl)méthyl]-3*H*-1,2,4-triazole-3-thione,
la 5-(2-chlorophényl)-1,2-dihydro-2-[(2-iodophényl)méthyl]-3*H*-1,2,4-triazole-3-thione,
la 5-(2-chlorophényl)-1,2-dihydro-2-[(2,5-diméthylphényl)méthyl]-3*H*-1,2,4-triazole-3-thione,
la 5-(2-chlorophényl)-1,2-dihydro-2-[(2,5-difluorophényl)méthyl]-3*H*-1,2,4-triazole-3-thione,
la 2-[(3-chlorophényl)méthyl]-5-(4-nitrophényl)-1,2-dihydro-3*H*-1,2,4-triazole-3-thione,
la 2-[(3-chlorophényl)méthyl]-5-(pyrimidin-5-yl)-1,2-dihydro-3*H*-1,2,4-triazole-3-thione,
la 5-(2-chlorophényl)-1,2-dihydro-2-[(3-cyanophényl)méthyl]-3*H*-1,2,4-triazole-3-thione,
la 5-(2-chlorophényl)-1,2-dihydro-2-[(4-nitrophényl)méthyl]-3*H*-1,2,4-triazole-3-thione,
la 5-(2-chlorophényl)-1,2-dihydro-2-(2-phényl)-éthyl-3*H*-1,2,4-triazole-3-thione,
la 5-(2-chlorophényl)-2-(2-(3-chlorophényl)éthyl)-1,2-dihydro-3*H*-1,2,4-triazole-3-thione,
la 5-(2-chlorophényl)-1,2-dihydro-2-(3-phényl)-propyl-3*H*-1,2,4-triazole-3-thione,
et leurs sels et solvates pharmaceutiquement acceptables.

5. Procédé de préparation d'un composé de formule (I) tel que défini dans la revendication 1, **caractérisé en ce qu'**il comprend :
(a) la mise en réaction d'un composé de formule générale (II), R¹-C(O)L, dans laquelle L représente un groupement partant et R¹ est tel que défini dans la formule (I), avec un composé de formule générale 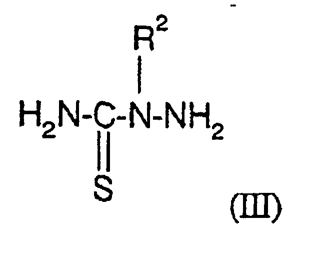 dans laquelle R² est tel que défini dans la formule (I), suivi de cyclisation ; ou
(b) la mise en réaction d'un composé de formule générale 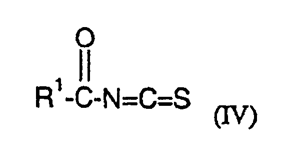 dans laquelle R¹ est tel que défini dans la formule (I), avec un composé de formule générale
R²-NHNH₂ (V)
dans laquelle R² est tel que défini dans la formule (I), suivi de cyclisation ; ou
(c) la mise en réaction d'un composé de formule générale 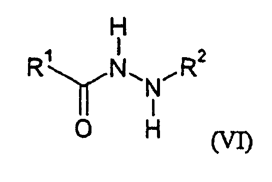 dans laquelle R¹ et R² sont tels que définis dans la formule (I), avec du thiocyanate d'ammonium, suivi de cyclisation ; ou
(d) la mise en réaction d'un composé de formule générale 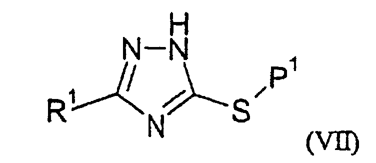
dans laquelle P¹ représente un groupement protecteur et R¹ est tel que défini dans la formule (I), avec un composé de formule générale (VIII), R²-L¹, dans laquelle L¹ représente un groupement partant et R² est tel que défini dans la formule (I), suivi de l'élimination du groupement protecteur P¹ ;
et éventuellement après (a), (b), (c) ou (d) la formation d'un sel ou solvate pharmaceutiquement acceptable du composé de formule (I).

6. Composé de formule (VII) telle que définie dans la revendication 5.

7. Composition pharmaceutique comprenant un composé de formule (I), ou un sel ou solvate pharmaceutiquement acceptable de celui-ci, selon l'une quelconque des revendications 1 à 4, en association avec un adjuvant, un diluant ou un support pharmaceutiquement acceptable.

8. Procédé de préparation d'une composition pharmaceutique selon la revendication 7, **caractérisé en ce qu'**il comprend le mélange d'un composé de formule (I), ou un sel ou solvate pharmaceutiquement acceptable de celui-ci, selon l'une quelconque des revendications 1 à 4, avec un adjuvant, un diluant ou un support pharmaceutiquement acceptable.

9. Composé de formule (I), ou un sel ou solvate pharmaceutiquement acceptable de celui-ci, selon l'une quelconque des revendications 1 à 4, destiné à être utilisé en thérapie.

10. Composé destiné à être utilisé selon la revendication 9, **caractérisé en ce que** la maladie est le psoriasis.
